# EUROPEAN PATENT APPLICATION

(11) **EP 4 226 906 A1**
(43) Date of publication of application: **16.08.2023**
(21) Application number: 21877676.3
(22) Date of filing: 06.10.2021
(51) Int. Cl.: A61H 5/00, A61B 10/00, A61B 3/113, G06F 3/01

(54) **SIGHTLINE-POSITION DETERMINATION DEVICE, SIGHTLINE-POSITION DETERMINATION METHOD, AND TRAINING METHOD**

(30) Priority: 07.10.2020 JP 2020169779; 07.06.2021 JP 2021095207
(71) Applicant: National University Corporation Chiba University, Chiba-shi, Chiba 263-8522 (JP); Sumitomo Pharma Co., Ltd., Osaka 541-0045 (JP)
(72) Inventor: NAKAGUCHI, Toshiya, Chiba-shi, Chiba 263-8522 (JP); SHIMIZU, Eiji, Chiba-shi, Chiba 260-8670 (JP); HIRANO, Yoshiyuki, Chiba-shi, Chiba 260-8670 (JP); SUTOH, Chihiro, Chiba-shi, Chiba 260-8670 (JP); SHIRAIWA, Naomi, Suita-shi, Osaka 564-0053 (JP); IKEDA, Yuki, Suita-shi, Osaka 564-0053 (JP); SAITO, Shunsuke, Osaka-shi, Osaka 541-0045 (JP)
(74) Representative: Vossius & Partner Patentanwälte Rechtsanwälte mbB
(86) International application number: PCT/JP2021/037044
(87) International publication number: WO 2022/075377

(57) **Abstract**

A sightline-position determination device includes: a sightline-position input unit to which a position of a sightline of a user is inputted; a determination region setting unit which sets a determination region; a determination unit which determines whether or not the position of the sightline inputted from the sightline-position input unit falls within the determination region; and an output unit which outputs feedback to the user when the determination unit has determined that the position of the sightline falls within the determination region.

## Description

### TECHNICAL FIELD

The present disclosure relates to a sightline-position determination device, a sightline-position determination system, a sightline-position determination method, a training system, an evaluating method, a training method, a monitoring method, a treating method, and a program.

### BACKGROUND ART

Currently, as one of effective options of treatment for neuropsychiatric disorders, for example, there has been executed Cognitive behavioral therapy as disclosed in NPL 1.

### PATENT DOCUMENT

### NON PATENT LITERATURE

NPL 1: Yoshinaga et al., Psychotherapy and Psychosomatics, 2016, 85(4), p.208-217.

### SUMMARY

### TECHNICAL PROBLEM

However, with the treating method in NPL 1, patients cannot have the treatment even if they desire to have the treatment, because of shortage of medical practitioner who can execute the treatment, in some cases.

It is an object of the present disclosure to provide a sightline-position determination device capable of reproducing a sightline communication environment close to the real society, a sightline-position determination system including the sightline-position determination device, a sightline-position determination method capable of reproducing a sightline communication environment close to the real society, a method for treating a neuropsychiatric disorder using the sightline-position determination method, a training system including the sightline-position determination device or the sightline-position determination system, a method for evaluating a sightline communication ability using the sightline-position determination method, a training method using the sightline-position determination method, a monitoring method using the sightline-position determination method, and a program for causing a computer to execute the sightline-determination position determination method or the training method.

### SOLUTION TO PROBLEM

A sightline-position determination device in one aspect of the present disclosure includes: a sightline-position input unit to which a position of a sightline of a user is inputted; a determination region setting unit which sets a determination region; a determination unit which determines whether or not the position of the sightline inputted from the sightline-position input unit falls within the determination region; and an output unit which outputs feedback to the user when the determination unit has determined that the position of the sightline falls within the determination region.

A sightline-position determination system in one aspect of the present disclosure includes the sightline-position determination device in the aforementioned aspect, and a sightline-position acquisition unit which acquires the position of the sightline of the user and outputs this position to the sightline-position input unit.

A sightline-position determination method in one aspect of the present disclosure includes: detecting a position of a sightline of a user; setting a determination region; determining whether or not the detected position of the sightline falls within the determination region; and outputting feedback to the user when it is determined that the position of the sightline falls within the determination region.

A training system in one aspect of the present disclosure includes the sightline-position determination device in any of the aforementioned aspect or the sightline-position determination system in any of the aforementioned aspect.

An evaluating method in one aspect of the present disclosure uses the sightline-position determination method in the aforementioned aspect.

A training method in one aspect of the present disclosure uses the sightline-position determination method in the aforementioned aspect.

A monitoring method in one aspect of the present disclosure uses the sightline-position determination method in the aforementioned aspect.

A treating method in one aspect of the present disclosure uses the training method in the aforementioned aspect.

A program in one aspect of the present disclosure causes a computer to execute the sightline-position determination method or the training method in the aforementioned aspects.

### ADVANTAGEOUS EFFECTS OF INVENTION

With the sightline-position determination device in the aforementioned aspect, it is possible to reproduce an environment of communication through a sightline close to the real society, for example, by setting the determination region such that it includes a social object to which a sightline is to be directed, and by performing the sightline coincidence determination and the feedback.

With the sightline-position determination system in the aforementioned aspects, it is possible to realize a sightline-position determination system capable of reproducing an environment of communication through a sightline close to the real society with the sightline-position determination device.

With the sightline-position determination method and the program in the aforementioned aspects, it is possible to reproduce an environment of communication through a sightline close to the real society, for example, by setting the determination region such that it includes a social object to which a sightline is to be directed, and by performing the sightline coincidence determination and the feedback.

With the treating method, the training method and the program in the aforementioned aspects, it is possible to reproduce an environment of communication through a sightline closer to the real society, through the sightline-position determination method, which makes it possible to realize treatment and training with high effects. In addition, it is possible to realize treatment and training which can be executed even in a situation where an executer (for example, a doctor, a psychologist, a counselor, or a therapist) is at a remote location or in a situation where the executer is absent.

With the training system in the aforementioned aspect, the sightline-position determination device or the sightline-position determination system and the sightline-position determination method reproduce an environment of communication through a sightline close to the real society, which can realize a system with a high training effect. In addition, it is possible to realize a training system which can be executed even in a situation where the executor is at a remote location or in a situation where the executor is absent.

With the evaluating method and the monitoring method in the aforementioned aspects, it is possible to realize a standardized evaluating method and a standardized monitoring method, through the sightline-position determination method, regardless of the expertise or proficiency of the executor, or even in a situation where the executor is remote or absent.

### BRIEF DESCRIPTION OF DRAWINGS

Fig. 1 is a block diagram illustrating the structure of a sightline-position determination system including a sightline-position determination device according to a first embodiment of the present disclosure.
Fig. 2 is a block diagram illustrating the structure of a sightline-position determination system including a sightline-position determination device according to a second embodiment of the present disclosure.
Fig. 3 is a block diagram illustrating a first modification of the sightline-position determination system in Fig. 1.
Fig. 4 is a block diagram illustrating a second modification of the sightline-position determination system in Fig. 1.
Fig. 5 is a block diagram illustrating the structure of a sightline-position determination system including a sightline-position determination device according to a third embodiment of the present disclosure.
Fig. 6 is a first view for explaining a target position and a determination region in the sightline-position determination device in Fig. 1.
Fig. 7 is a second view for explaining a target position and a determination region in the sightline-position determination device in Fig. 1.
Fig. 8 is a third view for explaining a target position and a determination region in the sightline-position determination device in Fig. 1.
Fig. 9 is a fourth view for explaining a target position and a determination region in the sightline-position determination device in Fig. 1.
Fig. 10 is a block diagram illustrating the structure of a sightline-position determination system including a sightline-position determination device according to a fourth embodiment of the present disclosure.
Fig. 11 is a flowchart for explaining sightline determination processing in the sightline-position determination device in Fig. 1.
Fig. 12 is a block diagram illustrating a third modification of the sightline-position determination system in Fig. 1.

### DETAILED DESCRIPTION

Hereinafter, an example of the present disclosure will be described with reference to the accompanying drawings.

### (First Embodiment)

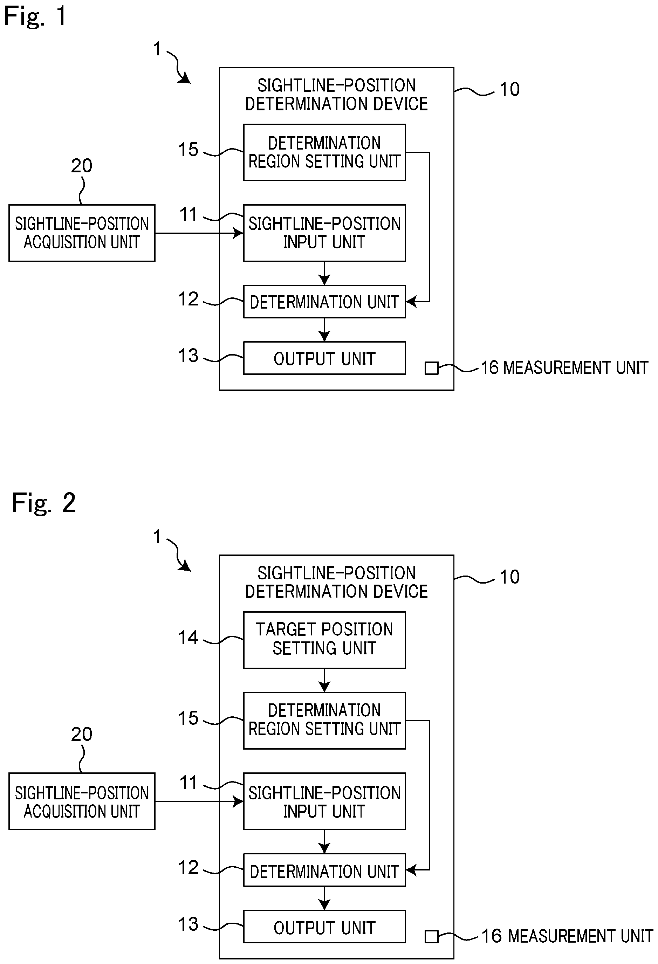

As illustrated in Fig. 1, a sightline-position determination system 1 according to a first embodiment of the present disclosure includes a sightline-position determination device 10 and a sightline-position acquisition unit 20. The sightline-position acquisition unit 20 acquires a position of a sightline of a user and outputs the acquired position to the sightline-position determination device 10.

As illustrated in Fig. 1, the sightline-position determination device 10 includes a sightline-position input unit 11, a determination region setting unit 15, a determination unit 12, and an output unit 13, and a CPU is caused to execute a predetermined program to realize their functions.

The position of the sightline of the user which has been outputted from the sightline-position acquisition unit 20 is inputted to the sightline-position input unit 11. The sightline-position input unit 11 outputs the inputted position of the sightline of the user to the determination unit 12.

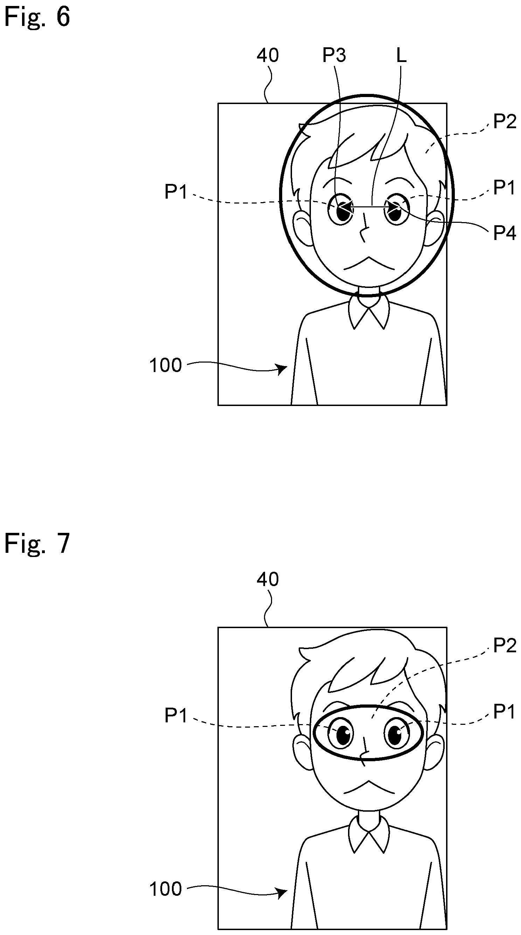

The determination region setting unit 15 sets a determination region P2 (see Fig. 6) such that it includes a target position P1 that is a position of a user's gaze target, and the determination region setting unit 15 outputs information about the determination region P2 to the determination unit 12. The determination region P2 is used as an allowable range of the position of the sightline in sightline coincidence determination. For example, by setting the determination region P2 such that it includes eyeballs of a person or animal to be gazed, it is possible to determine whether the user can make eye contact therewith.

For example, in a case where a plurality of target positions P1 are set, the determination region setting unit 15 can set a union of determination regions P2 set for the respective target positions P1 as a final determination region.

The target position P1 can be informed to the user by sound, as well as by an image. Therefore, the target position P1 and the determination region P2 may not be displayed in an image seen in a user's field of view (which will be referred to as a field-of-view image hereinafter). The target position P1 is set on a social object to which the user directs a sightline. For example, the target position P1 is set by preliminarily attaching temporal changes of X and Y coordinates to an image presented to the user (which will be referred to as a presentation image hereinafter). The social object may include, for example, a part of face, such as eyeballs, nose, eyebrows, mouth, etc., of a person or animal, a part of body, such as hair, ears, neck, shoulders, hands, etc., of a person or animal, an entire body of a person or animal, and all or a part of ornaments on body, including clothing, such as a tie or scarf.

For example, in assuming that the presentation image is presented to the user, the determination region setting unit 15 can set the determination region P2 depending on a size of an image of interest 100 based on a distance L between centers of right and left eyeballs (which will be referred to as eyeball positions P3 and P4, respectively, hereinafter) in a field-of-view image 40 illustrated in Fig. 6. More specifically, a size of the determination region P2 is defined with reference to a size of a face, and a distance L between centers of the right and left eyeballs is referred to as a factor for determining a size of the face.

A lower limit value can be provided to a size of the determination region P2. This is because it is difficult to accurately capture the position of the sightline of the user due to influences of involuntary eye movement of the eyeballs and detection noise of a sightline sensor when a screen presenting the presentation image thereon is too far from the user because the image of interest 100 in the presentation image or in the field-of-view image 40 is too small, for example.

The lower limit value of the determination region P2 may be an arbitrary value determined in advance regardless of the user, or may be an arbitrary value that can be set at the time of use. The lower limit value of the determination region P2 may be a value determined based on sightline-position variation data when the user gazes at an arbitrary point. For example, it is possible to use a region of a circle having a radius which is twice the standard deviation (2SD) of sightline-position variation data, as the lower limit value of the determination region P2. It is possible to expand a range of the region to three times the standard deviation (3SD) or four times the standard deviation (4SD), depending on a training stage. The lower limit value of the determination region P2 may be determined for each user or may be determined from an average value over a plurality of users. However, it is preferable that the lower limit value of the determination region P2 is determined for each user in consideration of variations in involuntary eye movement of eyeballs among individuals. As a default value for the sightline-position determination device 10, an average value over a plurality of healthy persons can be set as the lower limit value of the determination region P2. The sightline-position variation data means a plot of variation of the sightline position over time.

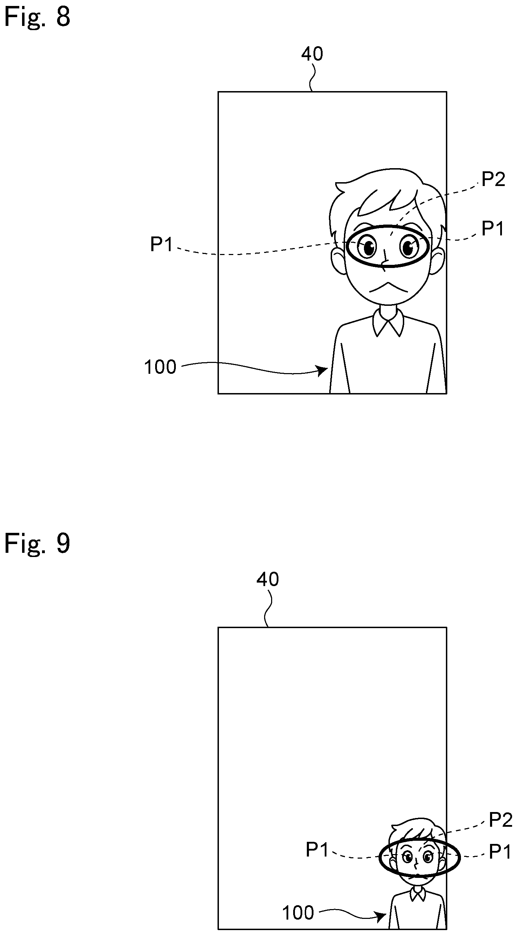

When the lower limit value is set to the determination region P2, the determination region P2 is set so as not to fall below the lower limit value. For example, when a size of the determination region P2 is calculated based on a distance L between the right and left eyeball positions P3 and P4 in the field-of-view image 40 and a size thereof is smaller than the lower limit value, the lower limit value is set as a size of the determination region P2. More specifically, for example, in a case where a size of the determination region P2 illustrated in Fig. 8 is the lower limit value, and the field-of-view image 40 is as in Fig. 9 in which the image of interest 100 is smaller than that in Fig. 8, the determination region P2 is set so as to have the same size as that in Fig. 8. This makes it possible to accurately determine whether the sightline is coincident without being affected by a size of an image of interest in a field-of-view image.

The determination unit 12 determines whether or not the position of the sightline of the user which has been outputted from the sightline-position input unit 11 falls within the determination region P2 (which will be referred to as sightline coincidence determination, hereinafter). The determination region P2 may be set in advance in the presentation image to be presented to the user at the time of the sightline coincidence determination, for example. The presentation image having the determination region P2 set therein may be stored in, for example, a storage apparatus (not illustrated) in the sightline-position determination device 10. The determination as to whether or not the position of the sightline of the user falls within the determination region P2 is performed as follows, for example.

First, the determination unit 12 determines whether or not the position of the sightline of the user has been located in the determination region P2. Then, the determination unit 12 determines whether or not time which has elapsed in the state where the position of the sightline of the user remains in the determination region P2 since the position of the sightline of the user got to be located in the determination region P2 (this time will be referred to as a first time, hereinafter) has reached a preset maintaining time (which is time for which the position of the sightline of the user should be maintained in the determination region P2 and will be referred to as a set maintaining time, hereinafter). If it is determined that the first time has reached the set maintaining time, the determination unit 12 determines that the position of the sightline of the user falls within the determination region P2 (in other words, the sightline is coincident). The first time may be the accumulated time for which the sightline of the user has remained in determination region P2 since the sightline of the user got to be located at first in determination region P2.

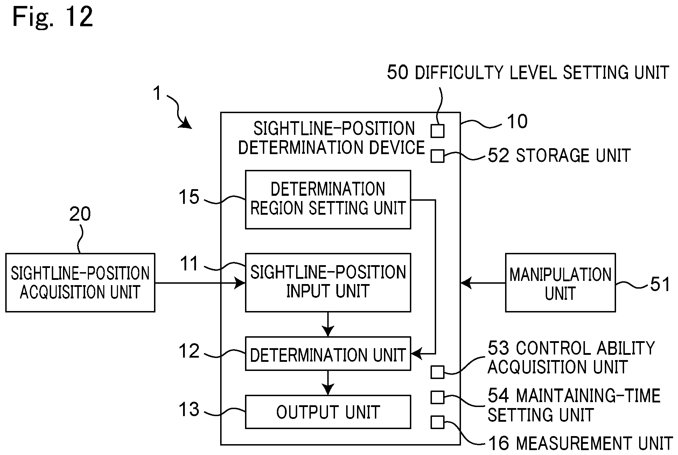

The first time is measured by, for example, a measurement unit 16 in the sightline-position determination device 10. The measurement unit 16 can further measure any one or more of the following indices for determining a social sightline control ability. For example, by further providing a storage unit 52 (see Fig. 12) for storing the position of the sightline of the user during training as a temporal transition of XY coordinates, it is possible to calculate a relationship between the position of the sightline of the user and the target position P1 or the determination region P2 through the XY coordinates, which makes it possible to measure various indices of the social sightline control ability. The indices for determining the social sightline control ability can be compiled for each task or can be compiled for the entire training. Examples of indices of the social sightline control ability will be described below.
·Whether or not the position of the sightline of the user has been located in the determination region P2
·An analyzed value of the first time (for example, an average value or a maximum value of the first time, and proportion of the first time (in other words, proportion of the time for which the position of the sightline of the user has remained in the determination region P2.))
·Time which has elapsed since the position of the sightline of the user was inputted to the sightline-position input unit at first until the position of the sightline of the user is determined to fall within the determination region P2 (this time will be referred to as a second time hereinafter)
·An average value of a distance between the target position P1 and the position of the sightline within a determination time (which will be referred to as a sightline distance hereinafter)
·The number of times the sightline has been determined to be coincident

The output unit 13 outputs feedback to the user, depending on whether or not the determination unit 12 has determined that the position of the sightline falls within the determination region P2. The feedback is given to the user and/or the person facing the user, using a sense selected from visual, auditory and tactile senses. The feedback is, for example, feedback constituted by a component selected from an image including a character, a symbol, and a painting, a sound including a voice, and vibration. The feedback is outputted through a display and/or a speaker (both of which are not illustrated), on or after it has been determined that the position of the sightline falls within the determination region P2 (for example, in real-time). The feedback may be outputted not only after it has been determined that the position of the sightline falls within the determination region P2 but also prior thereto. For example, a first-stage feedback can be outputted when the position of the sightline has been located in the determination region P2, and a second-stage feedback can be outputted when the first time has reached the set maintaining time. Furthermore, the output unit 13 can output feedback even when the position of the sightline is not located in the determination region P2. For example, the output unit 13 can output feedback for guiding the sightline toward the determination region P2. By outputting the feedback in multiple stages as described above, it is possible to enhance an effect of the training or the treatment. After completion of the training, the output unit 13 can output feedback regarding results of analyses of the indices of the social sightline control ability (for example, a result of the current training, a comparison between a result of the current training and a result of the past training, and a comparison between a result of the current training and an average value over other users), to the user, medical workers, a person facing the user, and the like. As the feedback regarding the results of analyses, a plurality of indices of the social sightline control ability may be individually outputted, or a plurality of indices of the social sightline control ability may be collectively outputted as a total score. An apparatus configured to output feedback may be separately provided, and feedback may be outputted through this apparatus. This apparatus may constitute a part of the sightline-position determination device 10, or may not constitute a part of the sightline-position determination device 10.

The respective units constituting the sightline-position determination device 10 may be provided in, for example, an external appliance (for example, a server). For example, in a case where the determination unit 12 is provided in an external appliance, the sightline-position determination device 10 further includes a communication apparatus connected to the external appliance in a wireless or wired manner. The sightline-position input unit 11 outputs the inputted position of the sightline of the user to the determination unit 12 in the external appliance through the communication apparatus. The output unit 13 acquires a result of determination by the determination unit 12 through the communication apparatus.

The sightline-position determination device 10 can also be structured as follows.

The sightline-position determination device 10 may be further provided with a difficulty level setting unit 50 (see Fig. 12), and the difficulty level setting unit 50 may be structured to change and set the difficulty level of the training (in other words, a load of the training). By incorporating the difficulty level setting unit 50 in the sightline-position determination device 10, it is possible to provide the training with a load suitable for the user. The difficulty level of the training includes at least one of a difficulty level of sightline coincidence determination and a difficulty level of a task or tasks. Examples of parameters of the difficulty level of sightline coincidence determination include a size of the determination region P2 and set maintaining time. A plurality of levels may be prepared for each parameter, and the difficulty level can be set by using one parameter at an arbitrary level or a combination of plural parameters at arbitrary levels. For example, as illustrated in Fig. 6, in a case where the determination region P2 is set to have a size including the entire face of a person and the set maintaining time is set to be 2 seconds, if the sightline coincides therewith, then the determination region P2 can be reduced as illustrated in Fig. 7, in order to raise the difficulty level of the sightline coincidence determination. If the sightline also coincides with the determination region P2 illustrated in Fig. 7, it is possible to further reduce the determination region P2 and/or increase the set maintaining time (to 3 seconds, for example), in order to further raise the difficulty level of the sightline coincidence determination.

The difficulty level setting unit 50 can be structured to set the difficulty level of the training based on the result of determination by the determination unit 12. For example, when the first time measured by the measurement unit 16 has reached the preset maintaining time, when it is determined that the position of the sightline falls within the determination region, the difficulty level setting unit 50 sets a load of the training by setting a size of the determination region and/or a length of the maintaining time. Specifically, when the determination unit 12 has not determined that the sightline is coincident, the difficulty level setting unit 50 can lower the difficulty level of sightline coincidence determination by enlarging the determination region P2 and/or reducing the maintaining time. By structuring the difficulty level setting unit 50 such that it adjusts the difficulty level of the training to a difficulty level suitable for the user, while not visualizing the determination region P2 for the user, it is possible to perform treatment or training without increasing user's awareness of difficulty and psychological burden on the user, which results in enhancement of the effect and continuity thereof. The sightline-position determination device 10 may be provided with a manipulation unit 51 (see Fig. 12) capable of receiving a difficulty level changing manipulation for changing the difficulty level of the training. The difficulty level setting unit 50 may be configured to set the difficulty level of the training based on the difficulty level changing manipulation received by the manipulation unit 51 or based on the social sightline control ability and/or an index for determining the social sightline control ability. In Fig. 12, the manipulation unit 51 is provided separately from the sightline-position determination device 10, but the manipulation unit 51 is not limited thereto, and may be provided integrally with the sightline-position determination device 10.

The sightline-position determination device 10 may be provided with a storage unit 52 (see Fig. 12) configured to store measured indices of the social sightline control ability. In this case, for example, when a value of the measured index of the social sightline control ability which has been measured by the measurement unit 16 is larger than a value of the index of the social sightline control ability which was measured by the measurement unit 16 before the measured index of the social sightline control ability and was stored in the storage unit 52, the difficulty level setting unit 50 increases a load of the training.

The sightline-position determination device 10 may be provided with a control ability acquisition unit 53 (see Fig. 12) configured to acquire indices of the social sightline control abilities of other users. In this case, for example, the difficulty level setting unit 50 makes comparison between a deviation of the index of the social sightline control ability of the user which was measured by the measurement unit 16 in each task with respect to an average value of the index of the social sightline control ability of the user, which was measured by the measurement unit 16, and a deviation of the index of the social sightline control ability of another user in each task with respect to an average value of the index of the social sightline control ability of the another user, which was acquired by the control ability acquisition unit 53, thereby estimating a task that is difficult for the user, and thereby the difficulty level setting unit 50 sets a load of the training based on the task estimated to be difficult for the user. This can enhance the effect of training. The index of the social sightline control ability of another user may be measured in advance by the measurement unit 16 and stored in the storage unit 52, or an external apparatus (for example, a server), for example. In this case, the control ability acquisition unit 53 acquires the index of the social sightline control ability of another user which are stored in the storage unit 52 or an external apparatus, through wireless or wired communication. The index of the social sightline control ability of another user may be measured by the measurement unit 16 in another sightline-position determination device 10, or may be measured by another apparatus than the sightline-position determination device 10. The task that is difficult for the user may be estimated by another component (for example, the control ability acquisition unit 53), instead of the difficulty level setting unit 50. In this case, the difficulty level setting unit 50 may be omitted, and the sightline-position determination device 10 may be configured not to set a load of the training.

The sightline-position determination device 10 may be provided with a maintaining-time setting unit 54 (see Fig. 12), and may be configured that the maintaining-time setting unit sets the set maintaining time.

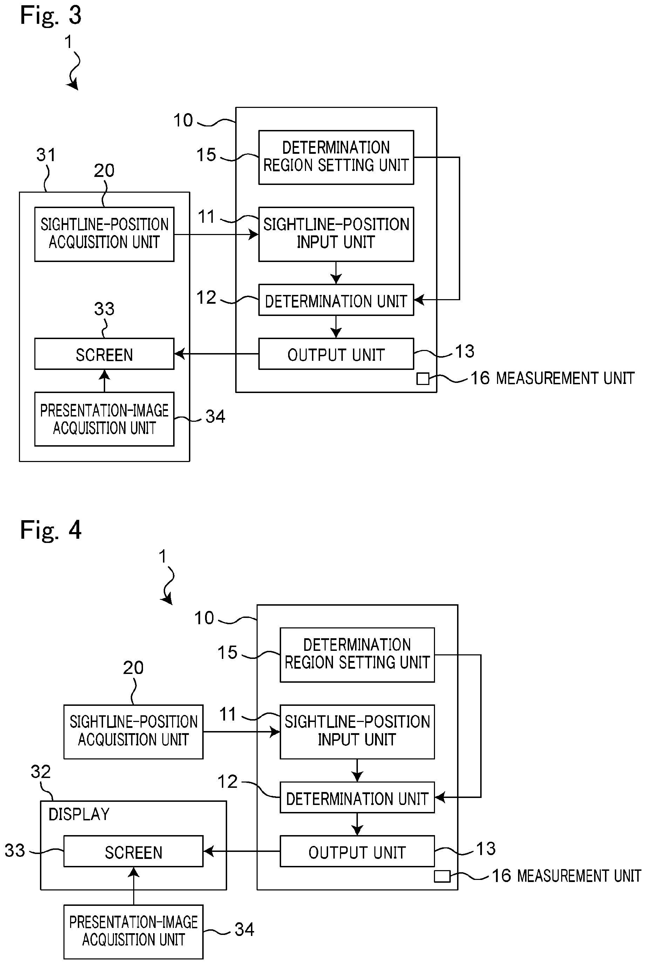

The sightline-position determination system 1 according to the first embodiment can be implemented in an aspect illustrated in Fig. 3 or 4, for example.

In the sightline-position determination system 1 in Fig. 3, for example, the sightline-position acquisition unit 20 (for example, an infrared-type sightline tracking sensor) is provided in a head mounted display (HMD) 31, and feedback outputted from the sightline-position determination device 10 is outputted through a screen 33, a speaker (not illustrated), or the like in the HMD 31. The presentation image is presented to the user through the screen 33 in the HMD 31, and the presentation image is regarded as the field-of-view image. The determination region P2 is set, for example, based on information about a target position P1 provided in advance on the presentation image. With the sightline-position determination system 1 in Fig. 3, due to the use of the HMD, it is possible to remove unnecessary external stimuli (disturbance), and to realize a higher sense of immersion. The HMD 31 is provided with a presentation-image acquisition unit 34 configured to acquire the presentation image. The presentation-image acquisition unit 34 may acquire the presentation image from an external apparatus connected thereto in a wireless or wired manner or from a storage apparatus (not illustrated) in the sightline-position determination device 10. In the sightline-position determination system 1 in Fig. 3, the sightline-position determination device 10 and the sightline-position acquisition unit 20 may be built in the HMD 31, or the sightline-position determination device 10 and the sightline-position acquisition unit 20 may be provided separately in the HMD 31.

In the sightline-position determination system 1 in Fig. 4, for example, the sightline-position determination device 10, the sightline-position acquisition unit 20 (for example, a sightline sensor), and the presentation-image acquisition unit 34 are provided separately, and feedback is outputted through a screen 33, a speaker (not illustrated), and the like in the display 32. The presentation image acquired by the presentation-image acquisition unit 34 is presented to the user through the screen 33 in the display 32, and the presentation image is regarded as a field-of-view image. The determination region P2 is set, for example, based on information about a target position P1 provided in advance on the presentation image. With the sightline-position determination system 1 in Fig. 4, it is possible to reduce a burden on the user, since the user wears nothing on his or her body. In the sightline-position determination system 1 in Fig. 4, the sightline-position acquisition unit 20 and the presentation-image acquisition unit 34 may be provided in the display 32.

The sightline-position determination system 1 may further include a display (not illustrated) for an executor for displaying, for a manipulator (an executor), the presentation image being presented to the user. The display for the executor displays the presentation image for the manipulator through a screen (not illustrated). The sightline-position determination system 1 may be configured to display the field-of-view image and the position of the sightline, in addition to the presentation image, through the display for the executor. The sightline-position determination system 1 may be configured to present the feedback outputted from the sightline-position determination device 10, through the screen, a speaker (not illustrated), or the like. The manipulator can grasp a result of the sightline position determination or progress of the training by looking at the display. This makes it possible to change the difficulty level of the training or to interrupt the training, through the manipulation unit 51, during the training.

The sightline-position determination device 10 may include any one or more or all of the measurement unit 16, the difficulty level setting unit 50, the manipulation unit 51, the storage unit 52, the control ability acquisition unit 53, and the maintaining-time setting unit 54.

### (Second Embodiment)

As illustrated in Fig. 2, a sightline-position determination system 1 according to a second embodiment of the present disclosure is different from that according to the first embodiment, in that a sightline-position determination device 10 includes a target position setting unit 14. In the second embodiment, the same parts as those in the first embodiment are denoted by the same reference numerals, and the description thereof will be omitted.

The target position setting unit 14 sets a target position P1 (see Fig. 6) which is a target to which the user of the sightline-position determination device 10 directs a sightline. The target position P1 is a position of a gaze target. For example, in a presentation image including an image (which will be referred to as an image of interest, hereinafter) of a person or animal to be gazed, at least one target position P1 is manually or automatically set at an arbitrary position on the image of interest. As a method for setting the target position, for example, it may use face recognition software, and/or an object detection algorithm, and/or the like.

Examples of the arbitrary position set as the target position P1 include an eyeball position, but the arbitrary position is not limited thereto. For example, a part of a face such as a nose, eyebrows, and a mouth may be set as the target position P1, a part of a body such as a hair, an ears, a neck, shoulders, and hands may be set as the target position P1, the image of interest 100 itself (the entire body of the person or animal) may be set as the target position P1, and a part of ornaments on a body including clothing such as a tie or a scarf may be set as the target position P1. The target position setting unit 14 may acquire the presentation image from a storage apparatus (not illustrated) in the sightline-position determination device 10, or from an external apparatus such as a server connected thereto in a wired or wireless manner.

### (Third Embodiment)

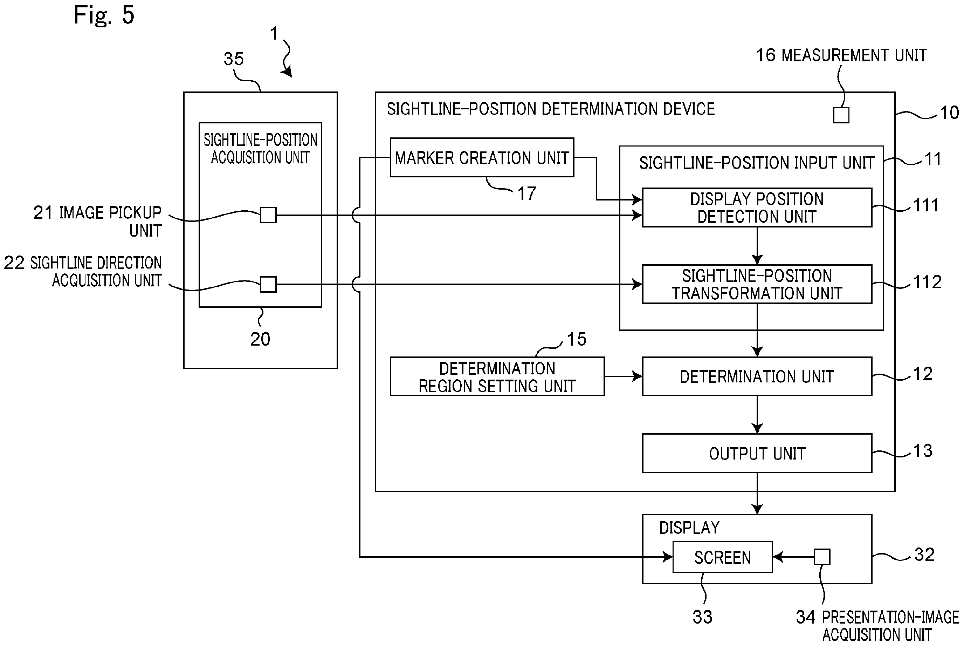

As illustrated in Fig. 5, a sightline-position determination system 1 according to a third embodiment of the present disclosure is different from that according to the first embodiment in the following points. In the third embodiment, the same parts as those in the first embodiment are denoted by the same reference numerals, and the description thereof will be omitted.
·A sightline-position determination device 10 further includes a marker creation unit 17.
·A sightline-position input unit 11 includes a display position detection unit 111 and a sightline-position transformation unit 112.
·A sightline-position acquisition unit 20 includes an image pickup unit 21 and a sightline direction acquisition unit 22.

In the third embodiment, for example, the sightline-position determination device 10 performs determination based on information from a wearable terminal 35 including the sightline-position acquisition unit 20, and feedback is outputted therefrom through a screen 33 or the like in a display 32.

The marker creation unit 17 creates a marker image which displays markers (for example, QR codes (registered trademark) and/or AR markers) at four corners of the screen 33, for example. The created marker image is outputted to the display position detection unit 111 in the sightline-position input unit 11 and to the screen 33. The marker image is created at the first time to use the sightline-position determination system 1, for example, and the marker image created first can be used thereafter. The marker image created first may be stored in a storage apparatus in the sightline-position determination device 10, or may be stored in a server connected thereto through a communication apparatus, for example.

The display position detection unit 111 receives the field-of-view image outputted from the image pickup unit 21 in the sightline-position acquisition unit 20 and the marker image created by the marker creation unit 17. The display position detection unit 111 detects a position of the marker image in the field-of-view image, calculates a coordinate transformation matrix (using a homography transformation matrix, for example), and outputs information about the matrix to the sightline-position transformation unit 112.

The sightline-position transformation unit 112 transforms the position of the sightline of the user in the coordinate system of the field-of-view image into a sightline position in the coordinate system of the presentation image, based on a sightline vector outputted from the sightline direction acquisition unit 22 in the sightline-position acquisition unit 20 and the information about the matrix which has been outputted from the display position detection unit 111. The transformed position of the sightline of the user is outputted to the determination unit 12.

The image pickup unit 21, which is an example of a field-of-view image acquisition unit, captures and acquires the field-of-view image of the user. The acquired field-of-view image is outputted to the display position detection unit 111. The sightline direction acquisition unit 22 detects a direction of the sightline (in other words, a sightline vector) of the user. The detected sightline vector is outputted to the sightline-position transformation unit 112.

With the sightline-position determination system 1 according to the third embodiment, it is possible to realize accurate sightline detection, regardless of a type of the display 32, due to the use of the wearable terminal including the sightline-position acquisition unit 20. It is also possible to store movements of the sightline outside the screen 33 and the user's head.

The sightline-position determination system 1 according to the third embodiment may be also provided with the target position setting unit 14 according to the second embodiment.

The marker creation unit 17 may be omitted. In this case, for example, a marker image (which will be referred to as a created marker image, hereinafter) is created outside the sightline-position determination system 1 and stored in an external apparatus such as a server. The created marker image is outputted from the external apparatus through a communication apparatus and is inputted to the display position detection unit 111.

### (Fourth Embodiment)

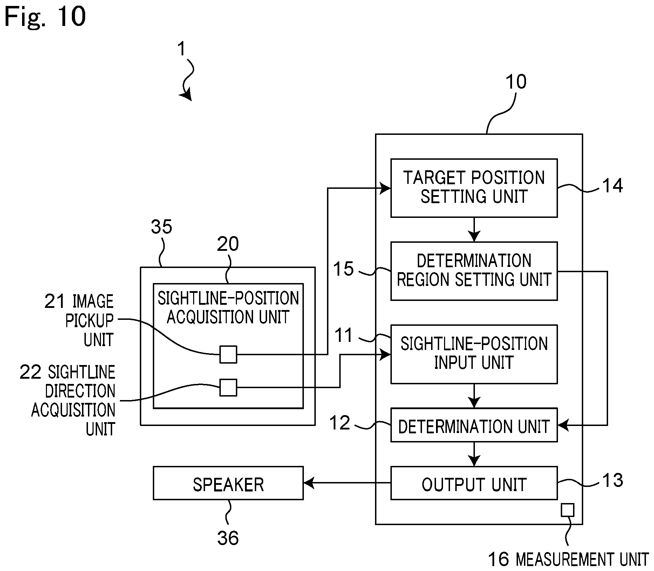

As illustrated in Fig. 10, a sightline-position determination system 1 according to a fourth embodiment of the present disclosure is different from that according to the second embodiment in the following points. In the fourth embodiment, the same parts as those in the second embodiment are denoted by the same reference numerals, and the description thereof will be omitted.
·A sightline-position acquisition unit 20 includes an image pickup unit 21 and a sightline direction acquisition unit 22.

In the fourth embodiment, the sightline-position acquisition unit 20 is provided in a wearable terminal 35, and feedback outputted from a sightline-position determination device 10 provided separately from the wearable terminal 35 is outputted as audio feedback through a speaker 36. In the fourth embodiment, the field-of-view image captured by the image pickup unit 21 is outputted to the target position setting unit 14. A target position P1 is set by the target position setting unit 14 and, thereafter, a determination region P2 is set based on information about the target position P1. The sightline direction acquisition unit 22 detects a direction of the sightline (the sightline vector) of the user, and the detected sightline vector is outputted to the sightline-position input unit 11.

With the sightline-position determination system 1 according to the fourth embodiment, the determination region P2 can be set so as to include a person facing the user, which enables experiencing realistic person-to-person communication. The feedback outputted from the sightline-position determination device 10 to the user is not limited to audio feedback, and may be outputted through a screen (not illustrated). The screen may be built in the wearable terminal 35 or may be provided separately from the wearable terminal 35.

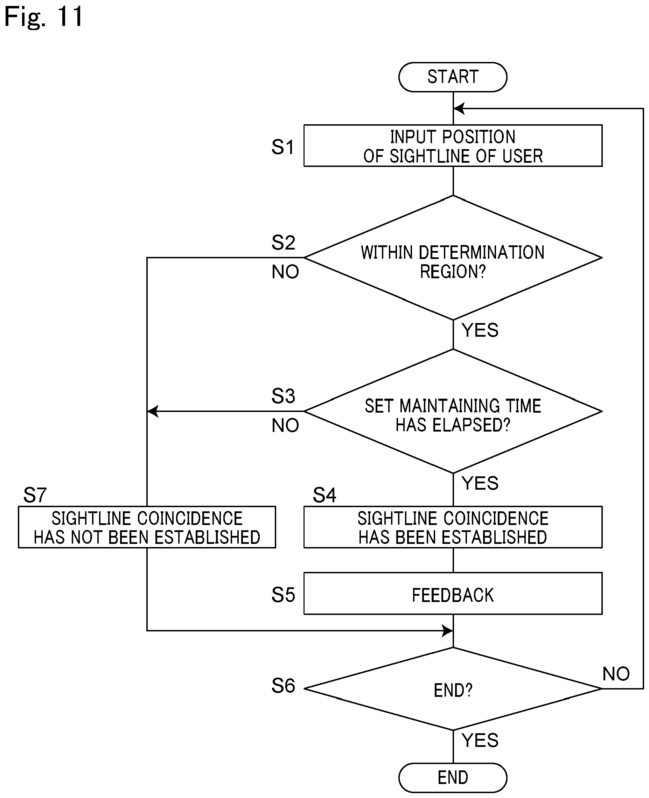

Next, there will be described a sightline-position determination method using the sightline-position determination device 10 (which will be referred to as a sightline-position determination method according to the present disclosure, hereinafter). For example, there is a sightline-position determination method in Fig. 11, and this sightline-position determination method is implemented by execution of a predetermined program by a CPU in the sightline-position determination device 10, for example. This sightline-position determination method may be applied to training and treatment for neuropsychiatric disorders.

When the sightline-position determination method is started, in the sightline-position determination device 10 (for example, the sightline-position determination devices 10 according to the second and fourth embodiments) which includes the target position setting unit 14, a target position P1 and a determination region P2 are set, and the difficulty level of the training is set. The setting of the difficulty level of the training and the maintaining time may be omitted in a case where they have been set in advance.

As illustrated in Fig. 11, a position of the sightline of the user is inputted to the sightline-position input unit 11 (step S1), and the determination unit 12 determines whether or not the position of the sightline of the user is located in the determination region P2 (step S2). When it is not determined that the position of the sightline is located in the determination region P2, the determination unit 12 determines that the sightline has not coincided (incoincidence of the sightline) (step S7).

When it is determined that the position of the sightline is located in the determination region P2, the determination unit 12 determines whether or not time elapsed in a state where the position of the sightline remains in the determination region P2 has reached a preset maintaining time (set maintaining time) (step S3). When it is not determined that the time elapsed in the state where the position of the sightline remains in the determination region P2 has reached the set maintaining time, the determination unit 12 determines that the sightline has not coincided (step S7).

When it is determined that the time elapsed in the state where the position of the sightline remains in the determination region P2 has reached the set maintaining time, the determination unit 12 determines that the sightline has coincided (step S4). Then, the output unit 13 outputs feedback to the user in real-time (step S5). The real-time includes a predetermined time lag (for example, a time lag which occurs for technical reasons). Thereafter, the sightline-position determination device 10 determines whether or not to end the sightline coincidence determination, in other words, whether or not to continuously perform the sightline coincidence determination (step S6). When it is not determined to end the sightline coincidence determination, in other words, when the sightline coincidence determination is to be continuously performed, this method returns to step S 1 where the position of the sightline of the user is inputted. When it is determined in step S6 to end the sightline coincidence determination, the sightline-position determination device 10 ends the sightline coincidence determination. When it is determined in step S7 that sightline coincidence determination is not established (namely, sightline coincidence is not established), this method can return to step S1 to perform the sightline coincidence determination again after lowering the difficulty level of the training. When the sightline coincidence determination has been successively unestablished a predetermined number of times or when a predetermined time has elapsed in a sightline incoincidence state, the sightline-position determination device 10 determines to end the sightline coincidence determination in step S6.

The sightline-position determination method using the sightline-position determination device 10 may be applied to, for example, diagnosis, severity classification, treatment, training, and/or monitoring for neuropsychiatric disorders. In the present disclosure, neuropsychiatric disorders include psychiatric disorders and neurodegenerative disorders.

Psychiatric disorders to which the present disclosure may be applied are not particularly limited, and include, for example, neurodevelopmental disorders, schizophrenia spectrum disorder and other psychotic disorders, anxiety disorders, obsessive-compulsive and related disorders, trauma- and stressor-related disorders, and neurocognitive disorders, according to the diagnostic criteria of DSM-5. Such psychiatric disorders include disorders and the like which tend to deteriorate the sightline communication ability along with psychiatric disorders. Such psychiatric disorders are not particularly limited, but preferably include neurodevelopmental disorders, anxiety disorders, obsessive-compulsive and related disorders, and trauma- and stressor-related disorders, and more preferably include neurodevelopmental disorders and anxiety disorders.

Disorders included in neurodevelopmental disorders are not particularly limited, and include, for example, intellectual disability, global developmental delay, language disorder, speech sound disorder, childhood-onset fluency disorder, social (pragmatic) communication disorder, unspecified communication disorders, autism spectrum disorder, attention-deficit hyperactivity disorder, specific learning disorder, developmental coordination disorder, stereotypic movement disorder, Tourette's disorder, persistent (chronic) motor or vocal tic disorder, provisional tic disorder, other specified tic disorder, unspecified tic disorder, other specified neurodevelopmental disorder, and unspecified neurodevelopmental disorder. Such disorders included in neurodevelopmental disorders are preferably language disorder, speech sound disorder, childhood-onset fluency disorder, social (pragmatic) communication disorder, unspecified communication disorder, autism spectrum disorder, attention-deficit hyperactivity disorder, specific learning disorder, stereotypic movement disorder, Tourette's disorder, persistent (chronic) motor or vocal tic disorder, provisional tic disorder, other specified tic disorder, unspecified tic disorder, other specified neurodevelopmental disorder, and unspecified neurodevelopmental disorder, and are more preferably childhood-onset fluency disorder and autism spectrum disorder.

Disorders included in schizophrenia spectrum and other psychotic disorders are not particularly limited, and include, for example, schizotypal personality disorder, delusional disorder, brief psychotic disorder, schizophreniform disorder, schizophrenia, schizoaffective disorder, substance/medication-induced psychotic disorder, psychotic disorder due to another medical condition, catatonia associated with another mental condition, catatonic disorder due to another medical condition, unspecified catatonia, other specified schizophrenia spectrum disorders and other psychotic disorders, unspecified schizophrenia spectrum disorders and other psychotic disorders, and attenuated psychosis syndrome (quasi-psychotic syndrome). Such disorders included in schizophrenia spectrum disorder and other psychotic disorders are preferably schizotypal personality disorder, schizophrenia, and schizoaffective disorder.

Disorders included in anxiety disorders are not particularly limited, and include, for example, separation anxiety disorder, selective mutism, specific phobia, social anxiety disorder, panic disorder, agoraphobia, generalized anxiety disorder, substance/medication-induced anxiety, anxiety disorder due to another medical condition, and other specified anxiety disorder. Such disorders included in anxiety disorders are preferably separation anxiety disorder, selective mutism, specific phobia, social anxiety disorder, panic disorder, agoraphobia, generalized anxiety disorder, and are more preferably social anxiety disorder.

Disorders included in obsessive-compulsive and related disorders, and trauma- and stressor-related disorders are not particularly limited, and include, for example, obsessive-compulsive disorder, body dysmorphic disorder, hoarding disorder, trichotillomania, excoriation disorder, substance/medication-induced obsessive-compulsive and related disorders, obsessive-compulsive and related disorders due to another medical condition, other specified obsessive-compulsive and related disorders, unspecified obsessive-compulsive and related disorder. Such disorders included in obsessive-compulsive and related disorders and trauma- and stressor-related disorders are preferably obsessive-compulsive disorder, body dysmorphic disorder, trichotillomania, excoriation disorder, and are more preferably body dysmorphic disorder.

Disorders included in trauma- and stressor-related disorders are not particularly limited, and include, for example, reactive attachment disorder, disinhibited social engagement disorder, posttraumatic stress disorder, acute stress disorder, adjustment disorders, other specified trauma- and stressor-related disorders, and unspecified trauma- and stressor-related disorder.

Disorders included in neurocognitive disorders are not particularly limited, and include, for example, delirium, dementia, and mild neurocognitive disorder. Such disorders included in neurocognitive disorders are preferably dementia, and mild neurocognitive disorder.

Neurodegenerative disorders are not particularly limited, and include, for example, Parkinson's disease, cranial neuropathy, and/or other disorders which tend to deteriorate the sightline communication ability.

The sightline-position determination method according to the present disclosure may also be applied to training of a person who has tended to deteriorate in sightline communication ability although not having been diagnosed as having a neuropsychiatric disorder, or a person who may have a difficulty in having social life due to deterioration of his or her sightline communication ability. This enables starting training at earlier timing, which can prevent neuropsychiatric disorders from being exacerbated and from being hard to treat.

Training using the sightline-position determination method according to the present disclosure (which will be referred to as training according to the present disclosure, hereinafter) is executed by a training system including the sightline-position determination device 10 or the sightline-position determination system 1. This training can reproduce an environment of communication using a sightline, which is close to the real society. For example, this training is executed with an aim of improving and enhancing the sightline communication ability. For example, by setting the determination region P2 such that it includes a social object based on the concept of Cognitive behavioral therapy, it is possible to execute various types of trainings of the sightline communication ability.

The sightline communication ability is an index of an ability to communicate through a sightline in an interpersonal relationship in the real society. The sightline communication ability is constituted by a social sightline control ability (an ability to control and use a sightline as a signals), a social sightline understanding ability (an ability to understand a sightline as a signal), a social sightline transmission ability (an ability to transmit a sightline as a signal in an appropriate context), a social sightline response ability (an ability to respond to a sightline as a signal in an appropriate context), and the like. Examples of the social sightline control ability include a social sightline coincidence ability, a social sightline shifting ability, and a conjugate gaze ability. These can be measured through training according to the present disclosure (see Table 1). It is possible to comprehensively evaluate the sightline communication ability at time of consultation while observing a relevance to clinical scores of the Liebowitz Social Anxiety Scale, the Social Phobia Inventory, the Patient Health Questionnaire -9, and the Generalized Anxiety Disorder-7. However, the sightline communication ability may also be evaluated based on any one of or a combination of two or more of indices for determining the social sightline control ability (namely, whether sightline coincidence is possible or impossible, an analyzed value of the first time, the second time, the sightline distance, and the number of times of sightline coincidence). For example, the sightline communication ability may be comprehensively evaluated at time of consultation by using a combination of a clinical score and an index for determining the social sightline control ability.

**[Table 1]**

| Index | Social sightline coincidence ability | Social sightline shifting ability | Conjugate gaze ability |
|---|---|---|---|
| Sightline coincidence is possible or impossible | Sightline coincidence is established or not established | Attention shift is established or not established | Sightline coincidence with sightline of another person or object pointed by another person (conjugate gaze) is established or not established |
| Analyzed value of first time | Duration time of sightline coincidence | - | Duration time of conjugate gaze |
| Second time | Time required for establishment of sightline coincidence | Time required for directing sightline to another social object | Time required for conjugate gaze |
| Sightline distance | Average value of distance between sightline and position of social object as gaze target | Average value of distance between sightline and object to which sightline is shifted | Average value of distance between sightline and object to be conjugately gazed |
| Number of times of sightline coincidence | Number of times sightline coincidence is established within a certain time period | Number of times attention shift is established within a certain time period | Number of times conjugate gaze is established within a certain time period |

In Table 1, the analyzed value of the first time is an average value or the maximum value of the time having elapsed in a state where the position of the sightline of the user remains in the determination region P2 since the position of the sightline of the user got to be located in the determination region P2, a proportion of the first time (the proportion of the time for which the position of the sightline of the user remains in the determination region P2). The second time is the time having elapsed since the position of the sightline of the user was inputted first to the sightline-position input unit until it is determined that the position of the sightline of the user falls within the determination region P2. The sightline distance is an average value of a distance between the target position P1 and the position of the sightline within a determination time.

Training for the social sightline control ability can be performed, for example, by setting the determination region P2 such that it includes a social object to which a sightline is to be directed. Training for the social sightline coincidence ability can be performed, for example, by setting the determination region P2 such that it includes a social object which is a gaze target. As an example, training for eye contact can be performed by setting the determination region P2 such that it includes eyeball positions. Training for the social sightline shifting ability can be performed, for example, by setting the determination region P2 such that it includes a social object to which a sightline is to be shifted. Training for the conjugate gaze ability can be performed, for example, by setting the determination region P2 such that it includes a social object specified as a common object by another person.

As previously described, the social sightline control ability is determined based on any one of or a combination of two or more whether or not the sightline can be coincident, the first time, the second time, the sightline distance, and the number of times the sightline coincides. For example, when different users tackle the same task, a user who has achieved sightline coincidence is determined to have higher social sightline control ability than a user who has not achieved sightline coincidence. Incidentally, the analyzed value of the first time being longer or larger, the second time being shorter, the sightline distance being shorter, the number of times the sightline coincides being larger, and the like mean that the social sightline control ability is higher.

The social sightline control ability may also be recorded over time to monitor treatment or training effects. For example, by causing the same user to repeat training, the same user can confirm that his or her social sightline control ability has been improved since the user has become able to perform sightline coincidence which the user was not able to perform before, the user has become able to extend or increase the analyzed value of the first time, the user has become able to shorten the second time, the user has become able to shorten the sightline distance, the user has become able to increase the number of times that the sightline coincides, and the like, in the same task. By performing training with an aim of improving the social sightline control ability, it is possible to expect improvement and enhancement of the sightline communication ability.

Examples of the social sightline transmission ability include "Power Gazing" indicating that the user himself or herself is in contact with another person with confidence, "Social Gazing" indicating that the user indicates an intention to have communication, and "Intimate Gazing" indicating that the user indicates an intention to be intimate with another person.

The training according to the present disclosure is constituted by an arbitrary task which provides one teaching for directing a sightline to a social object.

The task may be determined depending on the sightline communication ability to be improved. The task is not particularly limited provided that the task is formed for the purpose of improving and enhancing this ability, but it is preferable that viewpoints of Cognitive behavioral therapy are incorporated therein. By modifying a cognitively-and-behavioral biased pattern in the manner of Cognitive behavioral therapy, it is possible to obtain effects of attention shift training performed in Cognitive behavioral therapy, in addition to simply acquiring the social sightline control ability.

For example, patients with social anxiety disorder are in a state of inclining (biasing) their attention toward themselves (inside), and being unable to flexibly switch their attention. Therefore, by performing a task which provides a teaching for paying attention to recognition of facial expression or emotion of another person (for example, "Please look at the entire face of the partner. What kind of facial expression is it?" or "Please look at the entire face of the partner. How is the partner feeling !?"), it is possible to obtain an effect of facilitating the social sightline shift to the outside (another person or the like) in training for the social sightline shifting ability.

For example, patients with autism spectrum disorder are considered to have difficulties in "set sifting (cognitive flexibility)" and "central coherence (overall integrity)" which are cognitive tasks. Therefore, by performing a task which provides a teaching for looking at a part such as "Please look at both eyes" and a teaching for looking at the entire face such as "Please look at the entire face", interweavingly, it is possible to obtain an effect of enhancing "central coherence (overall integrity)", as well as "set shifting (cognitive flexibility)", in training for the social sightline shifting ability.

It is also possible to obtain the social sightline transmission ability by performing a task including a teaching for causing the user to indicate that he or she is being in contact with another person with confidence (for example, "Please look at the partner's forehead and both eyes alternately."), a teaching for causing the user to indicate his or her intention to have communication (for example, "Please look at the partner's both eyes and mouth alternately."), or a teaching for causing the user to indicate his or her intention to be intimate with another person (for example, "Please look at the partner's eyes and chest alternately.").

Conventionally, in Cognitive behavioral therapy, there has been formulated an auditory attention shift training method called "Attention Training Technique (ATT)", which includes distinguishing a plurality of sounds from each other by listening. The training according to the present disclosure is very advantageous in that this training can embody attention shift training using the visual sense by combining sightline coincidence determination performed by the training system according to the present disclosure with tasks modified in the cognitive behavioral manner.

Namely, the training may include a first task in which the determination region P2 is set such that it includes a constituent part (for example, the eyes, the nose, the mouth, the forehead, and the like) of a social object to which the sightline is to be directed, and a second task in which the determination region P2 is set such that it includes a constituent unit encompassing the constituent part (for example, when the eyes are a constituent part, the constituent unit is the face, the entire body, or the like). The training may also include a third task in which the determination region P2 is set such that it includes the constituent part and the constituent unit alternately, and a fourth task which causes the user to estimate the facial expression and/or emotion of the social object in which the determination region P2 is set.

The training is performed by causing the user to execute one or more tasks. For example, the training may be performed by execution of a plurality of tasks which are successively presented. A plurality of trainings may be performed as a series of trainings, at daily or hourly intervals. A result of the training can be calculated as evaluation for each task or comprehensive evaluation for a plurality of tasks. Each task is a unit of sightline coincidence determinations which are successively performed, each sightline coincidence determination being performed for a predetermined time (for example, 20 seconds or 30 seconds), and is configured in such a way as to obtain at least one result of sightline coincidence determination.

The difficulty level of the training may be arbitrarily determined. For example, the difficulty level of the training may be determined based on results of the trainings which were previously performed. The difficulty level of the training may be determined in such a way as to be gradually raised.

The difficulty level of the training can be set by the difficulty level of sightline coincidence determination and/or the difficulty level of a task.

As described above, the difficulty level of sightline coincidence determination may be set by the determination region P2 and/or the maintaining time. For example, the difficulty level of sightline coincidence determination may be changed by enlarging or reducing the determination region P2 and/or increasing or decreasing the maintaining time.

The difficulty level of a task is determined, for example, based on a subjective and/or objective evaluation of the user. The subjective and/or objective evaluation of the user includes, for example, a subjective and/or objective stress index of the user. The subjective stress of the user means a stress felt by the user, and examples of this stress index include a degree of anxiety about training or hardship of training which is reported by the user. The objective stress of the user means stress evaluated by a person other than the user, and examples of this stress index include a social sightline control ability evaluated during training, a heart rate and a heart-rate variation index during training, a degree of opening of the pupils during training, a blinking frequency during training, and changes thereof. Examples of the heart-rate variation index include a low frequency component (LF), a high frequency component (HF) and/or the ratio therebetween (LF/HF). The stress exerted on the user may be determined based on generally-expected liability to exert stress. For example, it can be said that a task constituted by a scene in which the user asks a person having a fearful appearance about a direction provides a situation which is more liable to exert stress on the user than a task constituted by a scene in which the user asks a person having a gentle appearance about a direction.

The difficulty level of a task is changed, for example, by changing elements constituting the task. Examples of elements constituting a task include, for example, those shown in Table 2, in addition to a teaching for directing a sightline to a social object.

**[Table 2]**

| Constituent element | Concrete example |
|---|---|
| Object | Animal, personified animal (living being intermediate between animal and human), human (male, female) |
| Age | Baby, child, adult, elderly person |
| Number of persons | One or plural |
| Facial expression, impression | Smiling face, angry face, expressionless face, good mood, bad mood, gentle, fearful |
| Scene | Home, workplace (meeting, interview, speech, self-introduction), commercial facilities (convenience store, supermarket, restaurant), asking directions in strange lands |
| Distance to object | Close, far, getting closer, getting far away |

As a method for presenting a task to the user, there are a method for presenting it using a video content (a moving image and/or a still image), a method for presenting it using a VR space, and a method for presenting it in the real world. The difficulty level of a task may be changed by a selected presenting method.

In a case of presenting a task using an image, the difficulty level of a task may be changed by a form of the image (a moving image, a still image) and a type of the image (an Illustration, an animation, a photograph).

With the training according to the present disclosure, it is possible to determine and change a task or tasks as described above, which makes it possible to provide a menu customized for each user, for example.

In a case of utilizing the training according to the present disclosure for treatment for a neuropsychiatric disorder, a time period of the training, a frequency of the training, and an execution time period per the single training are not particularly limited, and can be arbitrarily set by the user and/or the executor. Preferably, within a time period of 1 day to 1 year, the training is executed for 1 minute to 240 minutes per once, at a frequency of once per 90 days to 12 times per day. By performing the training for 1 minute only once per 90 days, it is possible to notice significance of sightline coincidence and/or eye contact with a social object, which raises expectation for effects of treatment. By intensively performing the training for 10 minutes once a day, 12 times in total, it is possible to raise expectation for effects of treatment. By performing the training at a slow pace of 20 minutes per once and once per 90 days, during 1 year, it is possible to raise expectation for effects of treatment. The execution time period per the single training may be arbitrarily set. However, in a case where the execution time period per the single training is less than 1 minute, there is a tendency to reduce the effect of treatment since the execution time period is too short. In a case where the execution time period per the single training exceeds 240 minutes, a load exerted on the user may be too large, which may reduce the effect of treatment. The frequency of execution of the training may be arbitrarily set. However, in a case of performing the training at a frequency lower than once per 90 days (for example, once per 120 days), intervals between the trainings is too long, which may reduce the effect of treatment. In a case of performing the training at a frequency higher than 12 times per day (for example, 20 times per day), a load exerted on the user may be too large, which may reduce the effect of treatment.

For example, in a case where the user visits a medical institution to undergo treatment, a time period of the raining, a frequency of the training, and an execution time period per the single training are not particularly limited, and can be arbitrarily set by the user and/or the executor. Preferably, the training is performed for 10 minutes to 40 minutes per once, 8 to 16 times in total, at a frequency of once or twice per week. This is because in consideration of the fact that the user visits a hospital about once a week in general, for example, assuming that the training for 20 minutes per once is performed once a week, it is considered that about 8 weeks are required to habituate the user thereto. In a case of performing treatment or the training outside a medical institution, for example, at home, a time period of the training, a frequency of the training, and an execution time period per the single training are not particularly limited, and can be arbitrarily set by the user. Preferably, the training for 10 minutes to 40 minutes per once is performed at a frequency of once a day or once a week. The total number of the trainings is not particularly limited, but it is preferable to perform the trainings in a range of 8 times to 30 times. It is also possible to use ambulatory treatment at a medical institution and home treatment in combination.

The training according to the present disclosure may also be applied to training of a person who has tended to deteriorate in sightline communication ability, or a person who may have a difficulty in having social life due to deterioration of his or her sightline communication ability, as well as a person having been diagnosed as having a neuropsychiatric disorder. In this case, similarly, a time period of the training, a frequency of training, and an execution time period per the single training are not particularly limited, and may be arbitrarily set by the user and/or the executor. The time period and the frequency are not particularly limited. Preferably, the training for 10 to 40 minutes per once is performed at a frequency of once a day or once a week.

With the sightline-position determination device 10 according to the present disclosure, it is possible to exhibit advantageous effects as follows.

The sightline-position determination device 10 according to the present disclosure includes the determination unit 12 configured to determine whether or not a position of a sightline of the user which has been inputted to the sightline-position input unit 11 falls within the determination region P2, the determination region setting unit 15 configured to set the determination region P2, and the output unit 13 configured to output feedback to the user when the determination unit 12 has determined that the position of the sightline falls within the determination region P2. For example, by setting the determination region P2 such that it includes eyeball positions of a person facing the user, it is possible to reproduce an environment of communication through sightline coincidence which is close to the real society. In the sightline-position determination device 10 according to the first embodiment, when it is determined that the position of the sightline of the user falls within the determination region, feedback is outputted to the user. Therefore, for example, in a case of using the sightline-position determination device 10 for treatment or training for a neuropsychiatric disorder, it is possible to increase willingness of the user to undergo treatment or training. For example, it is possible to perform various trainings for the sightline communication ability, by setting the determination region P2 based on the concept of Cognitive behavioral therapy.

The determination unit 12 determines that the position of the sightline falls within the determination region P2 when the time (the first time) having elapsed in the state where the position of the sightline of the user remains in the determination region P2 since the position of the sightline of the user got to be located in the determination region P2 reaches a preset maintaining time. With this structure, the sightline coincidence determination can be performed more accurately.

The measurement unit 16 measures indices of the social sightline control ability. For example, the measurement unit 16 measures, as indices of the social sightline control ability, at least one of an analyzed value of the first time, a second time having elapsed since the position of the sightline was inputted first to the sightline-position input unit 11 until the position of the sightline is determined to fall within the determination region P2, a distance between the position of the sightline of the user and the determination region P2, and the number of times it has been determined that the position of the sightline falls within the determination region P2. By using the measured indices, it is possible to determine the social sightline control ability of the user. For example, by recording the measured social sightline control ability over time, it is possible to diagnose neuropsychiatric disorders which make it hard to perform sightline control or it is possible to monitor an effects of treatment or training for neuropsychiatric disorders.

The output unit 13 outputs feedback to the user in real-time. With this structure, it is possible to reproduce an environment of communication through a sightline which is close to the real society.

The sightline-position determination device 10 further includes the target position setting unit 14 for setting a target position P1 to which the user directs a sightline. With this structure, for example, even in a case where a person facing the user moves, by setting the eyeball positions P3 and P4 to be the target position P1 in real-time, it is possible to reproduce an environment of communication through a sightline which is close to the real society.

The determination region setting unit 15 can set the determination region P2 such that it includes, for example, one selected from an entire body of a person facing the user, a part of a body of a person facing the user, an entire face of a person facing the user, a part of a face of a person facing the user, eye parts of a person facing the user, and a part of eyeball positions of the person facing the user. With this structure, it is possible to reproduce an environment of communication through a sightline which is close to the real society.

For example, the determination region setting unit 15 can set the determination region P2 as the target position P1 such that it includes one selected from an entire body of an animal including a person, a part of a body of an animal, an entire face of an animal, a part of a face of an animal, eye parts of an animal, and eyeball positions of an animal, in an image of the animal displayed on a display facing the user. With this structure, it is possible to reproduce an environment of communication through a sightline which is close to the real society.

For example, the determination region setting unit 15 can set the determination region P2 such that it includes eyeball positions of an animal in an image of the animal which includes a person facing the user or a person being displayed on the display facing the user. With this structure, it is possible to reproduce an environment of communication through a sightline which is close to the real society.

For example, the determination region setting unit 15 can set a size of the determination region P2 based on a distance L between left and right eyeball positions P3 and P4 of a person facing the user or a person being displayed on a display facing the user. With this structure, it is possible to calculate a size of the image of interest which is viewed from the user with a simple structure, and, for example, it is possible to use this size as an index of the difficulty level of a task or for standardization of a size of the determination region P2 among tasks.

A lower limit value is set to a size of the determination region P2. With this structure, it is possible to accurately capture the position of the sightline of the user, which makes it possible to perform sightline coincidence determination more accurately.

The sightline-position determination device 10 further includes the difficulty level setting unit 50 for setting the difficulty level of determination by the determination unit 12. With this structure, it is possible to set a plurality of difficulty levels for sightline coincidence determination.

The difficulty level setting unit 50 sets the difficulty level of sightline coincidence determination by setting a size of the determination region P2 and/or a length of the maintaining time. With this structure, it is possible to easily set a plurality of difficulty levels for sightline coincidence determination.

Feedback is performed using one or more senses selected from a visual sense, an auditory sense, and a tactile sense. With this structure, for example, in a case of using the sightline-position determination device 10 for treatment or training for a neuropsychiatric disorder, it is possible to increase the willingness of the user to undergo treatment or training.

The sightline-position determination system 1 according to the present disclosure includes the sightline-position determination device 10, and the sightline-position acquisition unit 20 configured to acquire the position of a sightline of the user and to output this position to the sightline-position input unit 11. With the sightline-position determination device 10, it is possible to realize the sightline-position determination system 1 capable of reproducing an environment of communication through a sightline which is close the real society.

The sightline-position determination method according to the present disclosure includes detecting a position of a sightline of the user, determining whether or not the detected position of the sightline falls within the determination region P2, and outputting feedback to the user when it is determined that the position of the sightline falls within the determination region P2. A program according to the present disclosure causes a computer to execute the sightline-position determination method according to the present disclosure. With this structure, for example, by setting the determination region P2 such that it includes a social object to which the sightline is to be directed and by performing sightline coincidence determination and feedback, it is possible to reproduce an environment of communication through a sightline which is closer to that in the real society. The sightline-position determination method according to the present disclosure may be applied to a method for diagnosing a neuropsychiatric disorder and a method for supporting a diagnosis of a neuropsychiatric disorder.

The sightline-position determination method according to the present disclosure includes successively presenting to the user a plurality of images each including a determination regions P2 set therein. With this structure, for example, by presenting images to the user in ascending order of the difficulty level of a task out of the plurality of images, it is possible to reproduce stepwise environments of communication through a sightline from communication through a sightline in an unreal society and to communication through a sightline which is close to the real society.

A treating method, a training method, an evaluating method, and a monitoring method according to the present disclosure use the sightline-position determination method according to the present disclosure. With the treating method and the training method according to the present disclosure, it is possible to reproduce an environment of communication through a sightline which is close to the real society easily and uniformly and to output feedback, through the sightline-position determination method, which can realize a treating method and a training method with high continuity and great effects. Effects of treatment with the treating method according to the present disclosure and effects of training with the training method according to the present disclosure mean expectation for improvement of at least any one of the following: the Liebowitz Social Anxiety Scale, the Social Phobia Inventory, the Patient Health Questionnaire-9, the Generalized Anxiety Disorder -7, the Health-Related QOL assessment (self-writing style) using the health economic Health-Related QOL Assessment Scale EuroQol 5 Dimension (EQ-5D), a feeling of anxiety about eye contact, and/or pleasure of eye contact. With the treating method and the training method according to the present disclosure, the user is enabled to adjust the difficulty levels of a task and determination, which can reduce anxiety and stress of the user. With the treating method and the training method according to the present disclosure, it is possible to provide standardized treatment and training, regardless of expertise and proficiency of an executor (for example, a doctor, a psychological specialist (psychologist), a counselor, and a therapist), even in a situation where the executor is at a remote location or in a situation where the executor is absent. This can reduce variations in scores of the sightline communication ability and the social sightline control ability from use day to use day, which enables using these scores as quantitative indices. Namely, with the evaluating method and the monitoring method according to the present disclosure, it is possible to realize an evaluating method and a monitoring method which enable quantitatively evaluating the sightline communication ability through the sightline-position determination method. The treating method according to the present disclosure also includes a treatment supporting method.

A training system according to the present disclosure includes the sightline-position determination device 10 or the sightline-position determination system 1, and is configured to be able to execute training using the sightline-position determination method. By the sightline-position determination device 10 or the sightline-position determination system 1 and the sightline-position determination method, it is possible to reproduce a communication environment close to the real society, thereby realizing an effective training system.

Various embodiments of the present disclosure have been described above in detail with reference to the drawings. Finally, various aspects of the present disclosure will be described.

A sightline-position determination device in a first aspect of the present disclosure includes: a sightline-position input unit configured to input a position of a sightline of a user to; a determination region setting unit configured to set a determination region; a determination unit configured to determine whether or not the position of the sightline inputted from the sightline-position input unit falls within the determination region; and an output unit configured to output feedback to the user when the determination unit has determined that the position of the sightline falls within the determination region.

In a second aspect of the present disclosure, the sightline-position determination device further includes a measurement unit configured to measure a first time having elapsed in a state where the position of the sightline remains in the determination region since the position of the sightline got to be located in the determination region, wherein the determination unit determines that the position of the sightline falls within the determination region, when the first time measured by the measurement unit has reached a preset maintaining time.

In a third aspect of the present disclosure, in the sightline-position determination device, the output unit outputs the feedback to the user in real-time.

In a fourth aspect of the present disclosure, the sightline-position determination device further includes a target position setting unit configured to set a target position to which the user directs a sightline.

In a fifth aspect of the present disclosure, in the sightline-position determination device, the determination region setting unit sets the determination region such that the determination region includes one selected from an entire body of an animal, which includes a person, facing the user, a part of the body of the animal facing the user, an entire face of the animal facing the user, a part of the face of the animal facing the user, an eye part of the animal facing the user, and a part of an eyeball position of the animal facing the user.

In a sixth aspect of the present disclosure, in the sightline-position determination device, the determination region setting unit sets the determination region such that the determination region includes one selected from an entire body of an animal including a person, a part of the body of the animal, an entire face of the animal, a part of the face of the animal, an eye part of the animal, and an eyeball position of the animal, in an image of the animal displayed on a display facing the user.

In a seventh aspect of the present disclosure, in the sightline-position determination device, the determination region setting unit sets the determination region such that the determination region includes an eyeball position of the animal.

In an eighth aspect of the present disclosure, in the sightline-position determination device, the determination region setting unit sets a size of the determination region, based on a distance between left and right eyeball positions of an animal, in an image of the animal, which includes a person, included in a field-of-view image of the user.

In a ninth aspect of the present disclosure, in the sightline-position determination device, a lower limit value is set to the size of the determination region.

In a tenth aspect of the present disclosure, in the sightline-position determination device, the animal is a human.

In an eleventh aspect of the present disclosure, in the sightline-position determination device, the measurement unit, which is configured to measure a first time having elapsed in a state where the position of the sightline remains in the determination region since the position of the sightline got to be located in the determination region, measures an index of a social sightline control ability of the user, in addition to the first time having elapsed in the state where the position of the sightline remains in the determination region since the position of the sightline got to be located in the determination region.

In a twelfth aspect of the present disclosure, in the sightline-position determination device, the index of the social sightline control ability is at least one of an analyzed value of the first time, a second time having elapsed since the position of the sightline was inputted first to the sightline-position input unit until the position of the sightline is determined to fall within the determination region, a distance between the position of the sightline of the user and the determination region, and a number of times it has been determined that the position of the sightline falls within the determination region.

In a thirteenth aspect of the present disclosure, the sightline-position determination device is configured to estimate a task which is difficult for the user, out of a plurality of tasks in training constituted by the plurality of tasks, by comparing the measured index of the social sightline control ability of the user with the index of the social sightline control ability of another user.

In a fourteenth aspect of the present disclosure, the sightline-position determination device further includes a difficulty level setting unit configured to set a load of training constituted by a plurality of tasks, wherein the difficulty level setting unit sets the load of the training based on the measured index of the social sightline control ability.

In a fourteenth-2 aspect of the present disclosure, the sightline-position determination device further includes a difficulty level setting unit configured to set a load of training constituted by a plurality of tasks.

In a fifteenth aspect of the present disclosure, in the sightline-position determination device, the difficulty level setting unit sets the load of the training based on a result of determination by the determination unit.

In a sixteenth aspect of the present disclosure, in the sightline-position determination device, the difficulty level setting unit increases the load of the training, when a value of the measured index of the social sightline control ability is greater than a value of the index of the social sightline control ability which was measured before the measured index of the social sightline control ability.

In a sixteenth-2 aspect of the present disclosure, the sightline-position determination device further includes a measurement unit configured to measure a first time having elapsed in a state where the position of the sightline remains in the determination region since the position of the sightline got to be located in the determination region, wherein the determination unit determines that the position of the sightline falls within in the determination region, when the first time measured by the measurement unit has reached a preset maintaining time, and the difficulty level setting unit sets the load of the training, by setting a size of the determination region and/or a length of the maintaining time.

In a seventeenth aspect of the present disclosure, in the sightline-position determination device, the difficulty level setting unit estimates the task which is difficult for the user, by making a comparison between a deviation of the measured index of the social sightline control ability of the user in each of the tasks with respect to an average value of the measured index of the social sightline control ability of the user, and a deviation of the index of the social sightline control ability of another user in each of the tasks with respect to an average value of the index of the social sightline control ability of the another user, and the difficulty level setting unit sets the load of the training based on the estimated task.

In a seventeenth-2 aspect of the present disclosure, the sightline-position determination device further includes a measurement unit configured to measures a first time having elapsed in a state where the position of the sightline remains in the determination region since the position of the sightline got to be located in the determination region, wherein the measurement unit measures an index of the social sightline control ability of the user, in addition to the first time.

In an eighteenth aspect of the present disclosure, the sightline-position determination device further includes a measurement unit configured to measure a first time having elapsed in a state where the position of the sightline remains in the determination region since the position of the sightline got to be located in the determination region, wherein the determination unit determines that the position of the sightline falls within the determination region, when the first time measured by the measurement unit has reached a preset maintaining time, and the difficulty level setting unit sets the load of the training, by setting a size of the determination region and/or a length of the maintaining time.

In an eighteenth-2 aspect of the present disclosure, in the sightline-position determination device, the index of the social sightline control ability is at least one of an analyzed value of the first time, a second time having elapsed since the position of the sightline was inputted first to the sightline-position input unit until the position of the sightline is determined to fall within the determination region, a distance between the position of the sightline of the user and the determination region, and a number of times it has been determined that the position of the sightline falls within the determination region.

In a nineteenth aspect of the present disclosure, the sightline-position determination device further includes a manipulation unit capable of receiving a difficulty level changing manipulation for changing the load of the training, wherein the difficulty level setting unit sets a difficulty level of the training based on the difficulty level changing manipulation received by the manipulation unit.

In a nineteenth-2 aspect of the present disclosure, in the sightline-position determination device, the difficulty level setting unit increases the load of the training, when a value of the measured index of the social sightline control ability is greater than a value of the index of the social sightline control ability which was measured before the measured index of the social sightline control ability.

In a twentieth aspect of the present disclosure, in the sightline-position determination device, the feedback is performed using one or more senses selected from a visual sense, an auditory sense, and a tactile sense.

In a twentieth-2 aspect of the present disclosure, in the sightline-position determination device, the difficulty level setting unit estimates the task which is difficult for the user, by making a comparison between a deviation of the measured index of the social sightline control ability of the user in each of the tasks with respect to an average value of the measured index of the social sightline control ability of the user, and a deviation of the measured index of the social sightline control ability of another user in each of the tasks with respect to an average value of the index of the social sightline control ability of the another user, and the difficulty level setting unit sets the load of the training based on the estimated task.

In a twenty-first aspect of the present disclosure, the sightline-position determination device is used for diagnosis, severity classification, treatment, training and/or monitoring of a neuropsychiatric disorder or for enhancement of a sightline communication ability.

In a twenty-first-2 aspect of the present disclosure, the sightline-position determination device further includes a manipulation unit capable of receiving a difficulty level changing manipulation for changing the load of the training, wherein the difficulty level setting unit sets the difficulty level of the training based on the difficulty level changing manipulation received by the manipulation unit.

In a twenty second aspect of the present disclosure, in the sightline-position determination device, the neuropsychiatric disorder is included in anxiety disorders or neurodevelopmental disorders.

A sightline-position determination system in a twenty third aspect of the present disclosure includes: the sightline-position determination device in any of the aforementioned aspects; and a sightline-position acquisition unit which includes a sightline direction acquisition unit configured to detect a direction of a sightline of the user, the sightline-position acquisition unit being configured to output the direction of the sightline of the user which has been detected by the sightline direction acquisition unit, to the sightline-position input unit.

In a twenty fourth aspect of the present disclosure, in the sightline-position determination system, the sightline-position acquisition unit further includes a field-of-view image acquisition unit configured to acquire a field-of-view image of the user, the field-of-view image acquisition unit being configured to acquire a position of the sightline of the user based on the field-of-view image.

In a twenty fifth aspect of the present disclosure, in the sightline-position determination system, the sightline-position input unit includes a display position detection unit configured to detect a position of a presentation image presented to the user in the field-of-view image acquired by the field-of-view image acquisition unit, and a sightline-position transformation unit configured to transform the position of the sightline of the user in a coordinate system of the field-of-view image into a position of the sightline of the user in a coordinate system of the presentation image, based on the detected position of the presentation image.

A sightline-position determination method in a twenty sixth aspect of the present disclosure includes: inputting a position of a sightline of a user; setting a determination region; determining whether or not the inputted position of the sightline falls within the determination region; and outputting feedback to the user when it is determined that the position of the sightline falls within the determination region.

In a twenty seventh aspect of the present disclosure, the sightline-position determination method further includes measuring a first time having elapsed in a state where the position of the sightline remains in the determination region since the position of the sightline got to be located in the determination region, and determining that the position of the sightline falls within the determination region, when the measured first time has reached a preset maintaining time.

In a twenty eighth aspect of the present disclosure, in the sightline-position determination method, the feedback is outputted to the user in real-time.

In a twenty ninth aspect of the present disclosure, the sightline-position determination method further includes setting a target position to which the user directs s sightline, and setting the determination region including the target position.

In a thirtieth aspect of the present disclosure, in the sightline-position determination method, the determination region is set such that the determination region includes one selected from an entire body of an animal, which includes a person, facing the user, a part of the body of the animal facing the user, an entire face of the animal facing the user, a part of the face of the animal facing the user, an eye part of the animal facing the user, and a part of an eyeball position of the animal facing the user.

In a thirty first aspect of the present disclosure, in the sightline-position determination method, the determination region is set such that the determination region includes one selected from an entire body of an animal including a person, a part of the body of the animal, an entire face of the animal, a part of the face of the animal, an eye part of the animal, and an eyeball position of the animal, in an image of the animal displayed on a display facing the user.

In a thirty second aspect of the present disclosure, in the sightline-position determination method, a size of the determination region is set based on a distance between left and right eyeball positions of an animal including a person facing the user.

In a thirty third aspect of the present disclosure, in the sightline-position determination method, a lower limit value is set to the size of the determination region.

In a thirty fourth aspect of the present disclosure, in the sightline-position determination method, the lower limit value is determined based on sightline-position variation data representing a sightline position which varies with time when the user gazes at an arbitrary point.

In a thirty fifth aspect of the present disclosure, in the sightline-position determination method, the animal is a human.

A training system in a thirty sixth aspect of the present disclosure includes the sightline-position determination device in any of the aforementioned aspects or the sightline-position determination system in any of the aforementioned aspects.

An evaluating method in a thirty seventh aspect of the present disclosure uses the sightline-position determination method in any of the aforementioned aspects.

In a thirty eighth aspect of the present disclosure, in the evaluating method, the sightline communication ability is a social sightline control ability or a social sightline transmission ability.

In a thirty ninth aspect of the present disclosure, in the evaluating method, the social sightline control ability is a social sightline coincidence ability, a social sightline shifting ability, or a conjugate gaze ability.

A training method in a fortieth aspect of the present disclosure includes executing training using the sightline-position determination method in any of the aforementioned aspects.

In a forty first aspect of the present disclosure, in the training method, the training is constituted by a plurality of tasks structured to obtain at least one result of determination as to whether or not the position of the sightline falls within the determination region, and the plurality of tasks are successively executed, and the tasks include directing a sightline to one of a plurality of the determination regions, according to a teaching.

In a forty second aspect of the present disclosure, in the training method, the tasks include a first task in which the determination region is set such that the determination region includes a constituent part of a social object to which a sightline is to be directed; and a second task in which the determination region is set such that the determination region includes a constituent unit encompassing the constituent part.

In a forty third aspect of the present disclosure, in the training method, the tasks include a third task in which the determination region is set such that the determination region includes the constituent part and the constituent unit, alternately.

In a forty fourth aspect of the present disclosure, in the training method, the tasks include a fourth task of estimating a facial expression and/or an emotion of a social object in which the determination region is set, in addition to directing a sightline to the determination region.

In a forty fifth aspect of the present disclosure, in the training method, a load of the training is set by a difficulty level of the tasks.

In a forty sixth aspect of the present disclosure, in the training method, a load of the training is set based on an index including subjective or objective evaluation of the user.

In a forty seventh aspect of the present disclosure, in the training method, the subjective evaluation of the user includes an evaluation, which is self-reported by the user, of hardship which was felt actually by the user executing the training.

In a forty-seventh-2 aspect of the present disclosure, in the training method, a load of the training is set according to a result of determination as to whether or not the position of the sightline falls within the determination region.

In a forty eighth aspect of the present disclosure, in the training method, the objective evaluation of the user includes at least one of a result of the training, a heart rate change and/or a heart-rate variation index of the user during the training, a degree of opening of pupils of the user, and a change of a blinking frequency of the user.

In a forty-eighth-2 aspect of the present disclosure, in the training method, when a first time having elapsed in a state where the position of the sightline remains in the determination region since the position of the sightline got to be located in the determination region has reached a preset maintaining time, and it is determined that the position of the sightline falls within in the determination region, the load of the training is set by setting a size of the determination region and/or a length of the maintaining time.

In a forty ninth aspect of the present disclosure, the training method includes measuring an index of a social sightline control ability which is an ability to control and use a sightline as a signal.

In a fiftieth aspect of the present disclosure, in the training method, the index of the social sightline control ability is at least one of a first time having elapsed in a state where the position of the sightline remains in the determination region since the position of the sightline got to be located in the determination region, a second time having elapsed since the position of the sightline was inputted first until the position of the sightline is determined to fall within the determination region, a distance between the position of the sightline of the user and the determination region, and a number of times it has been determined that the position of the sightline falls within the determination region.

In a fifty first aspect of the present disclosure, in the training method, a load of the training is set according to the measured index of the social sightline control ability of the user.

In a fifty second aspect of the present disclosure, in the training method, a load of the training is set according to a result of determination as to whether or not the position of the sightline falls within the set determination region.

In a fifty third aspect of the present disclosure, in the training method, when the first time has reached a preset maintaining time, and it is determined that the position of the sightline falls within the determination region, a load of the training is set by setting a size of the determination region and/or a length of the maintaining time.

In a fifty-third-2 aspect of the present disclosure, the training method includes measuring a first time having elapsed in a state where the position of the sightline remains in the determination region since the position of the sightline got to be located in the determination region, and an index of a social sightline control ability which is an ability to control and use a sightline as a signal.

In a fifty fourth aspect of the present disclosure, in the training method, the training is structured in such a way as to present, to the user, tasks successively in ascending order of a difficulty level, out of a plurality of tasks structured to obtain at least one result of determination as to whether or not the position of the sightline falls within the determination region.

In a fifty-fourth-2 aspect of the present disclosure, in the training method, the index of the social sightline control ability is at least one of an analyzed value of the first time, a second time having elapsed since the position of the sightline was inputted first until the position of the sightline is determined to fall within the determination region, a distance between the position of the sightline of the user and the determination region, and a number of times it has been determined that the position of the sightline falls within the determination region.

In a fifty-fifth-2 aspect of the present disclosure, the training method includes increasing a load of the training, when a value of the measured index of the social sightline control ability is greater than a value of the index of the social sightline control ability which was measured before the measured index of the social sightline control ability.

In a fifty-sixth-2 aspect of the present disclosure, the training method includes estimating the task which is difficult for the user, by making a comparison between a deviation of the measured index of the social sightline control ability of the user in each of the tasks with respect to an average value of the measured index of the social sightline control ability of the user, and a deviation of the index of the social sightline control ability of another user in each of the tasks with respect to an average value of the index of the social sightline control ability of the another user, and setting a load of the training based on the estimated task.

In a fifty-seventh-2 aspect of the present disclosure, the training method includes setting a difficulty level of the training based on a difficulty level changing manipulation for changing the load of the training.

A monitoring method in a fifty fifth aspect of the present disclosure uses the sightline-position determination method in any of the aforementioned aspects.

A method for treating a neuropsychiatric disorder in a fifty sixth aspect of the present disclosure includes executing training using the training method in any of the aforementioned aspects.

In a fifty seventh aspect of the present disclosure, in the method for treating a neuropsychiatric disorder, the training is executed for 1 minute to 240 minutes per once, at a frequency of once per 90 days to 12 times per day, within a time period of 1 day to 1 year.

In a fifty eighth aspect of the present disclosure, in the method for treating a neuropsychiatric disorder, a load of the training is increased stepwisely.

In a fifty ninth aspect of the present disclosure, in the method for treating a neuropsychiatric disorder, a difficulty level of the training is changed depending on the user.

A method for diagnosing a neuropsychiatric disorder in a sixtieth aspect of the present disclosure uses the sightline-position determination method in any of the aforementioned aspects.

A program in a sixty first aspect of the present disclosure causes a computer to execute the sightline-position determination method in any of the aforementioned aspects.

A program in a sixty second aspect of the present disclosure causes a computer to execute the training method in any of the aforementioned aspects.

A sightline-position determination device in an A-first aspect of the present disclosure includes: a sightline-position input unit configured to input a position of a sightline of a user; a determination region setting unit configured to set a determination region; a determination unit configured to determine whether or not the position of the sightline inputted from the sightline-position input unit falls within the determination region; and an output unit configured to output feedback to the user when the determination unit has determined that the position of the sightline falls within the determination region.

In an A-second aspect of the present disclosure, the sightline-position determination device further includes a measurement unit configured to measure a first time having elapsed in a state where the position of the sightline remains in the determination region since the position of the sightline got to be located in the determination region, wherein the determination unit determines that the position of the sightline falls within in the determination region, when the first time measured by the measurement unit has reached a preset maintaining time.

In an A-third aspect of the present disclosure, in the sightline-position determination device, the measurement unit measures an index of a social sightline control ability, in addition to the first time.

In an A-fourth aspect of the present disclosure, in the sightline-position determination device, the index of the social sightline control ability is at least one of an analyzed value of the first time, a second time having elapsed since the position of the sightline was inputted first to the sightline-position input unit until the position of the sightline is determined to fall within the determination region, a distance between the position of the sightline of the user and the determination region, and a number of times it has been determined that the position of the sightline falls within the determination region.

In an A-fifth aspect of the present disclosure, in the sightline-position determination device, the output unit outputs the feedback to the user in real-time.

In an A-sixth aspect of the present disclosure, the sightline-position determination device further includes a target position setting unit configured to set a target position to which the user directs a sightline.

In an A-seventh aspect of the present disclosure, in the sightline-position determination device, the determination region setting unit sets the determination region such that the determination region includes one selected from an entire body of an animal, which includes a person, facing the user, a part of the body of the animal facing the user, an entire face of the animal facing the user, a part of the face of the animal facing the user, an eye part of the animal facing the user, and a part of an eyeball position of the animal facing the user.

In an A-eighth aspect of the present disclosure, in the sightline-position determination device, the determination region setting unit sets the determination region such that the determination region includes one selected from an entire body of an animal including a person, a part of the body of the animal, an entire face of the animal, a part of the face of the animal, an eye part of the animal, and an eyeball position of the animal, in an image of the animal displayed on a display facing the user.

In an A-ninth aspect of the present disclosure, in the sightline-position determination device, the determination region setting unit sets the determination region such that the determination region includes an eyeball position of the animal.

In an A-tenth aspect of the present disclosure, in the sightline-position determination device, the determination region setting unit sets a size of the determination region, based on a distance between left and right eyeball positions of an animal, in an image of the animal, which includes a person, included in a field-of-view image of the user.

In an A-eleventh aspect of the present disclosure, in the sightline-position determination device, a lower limit value is set to the size of the determination region.

In an A-twelfth aspect of the present disclosure, in the sightline-position determination device, the animal is a human.

In an A-thirteenth aspect of the present disclosure, the sightline-position determination device further includes a difficulty level setting unit configured to set a difficulty level of training constituted by a plurality of tasks, wherein the difficulty level of the training is set by a difficulty level of the tasks and/or a difficulty level of sightline coincidence determination which is determination as to whether or not the inputted position of the sightline falls within the determination region.

In an A-fourteenth aspect of the present disclosure, the sightline-position determination device further includes a measurement unit configured to measure a first time having elapsed in a state where the position of the sightline remains in the determination region since the position of the sightline got to be located in the determination region, wherein the determination unit determines that the position of the sightline falls within the determination region, when the first time measured by the measurement unit has reached a preset maintaining time, and the difficulty level setting unit sets the difficulty level of the sightline coincidence determination, by setting a size of the determination region and/or a length of the maintaining time.

In an A-fifteenth aspect of the present disclosure, in the sightline-position determination device, the difficulty level setting unit sets the difficulty level of the training based on a result of determination by the determination unit.

In an A-sixteenth aspect of the present disclosure, the sightline-position determination device further includes a manipulation unit capable of receiving a difficulty level changing manipulation for changing the difficulty level of the training, wherein the difficulty level setting unit sets the difficulty level of the training based on the difficulty level changing manipulation received by the manipulation unit.

In an A-seventeenth aspect of the present disclosure, in the sightline-position determination device, the feedback is performed using one or more senses selected from a visual sense, an auditory sense, and a tactile sense.

In an A-eighteenth aspect of the present disclosure, the sightline-position determination device is used for diagnosis, severity classification, treatment, training and/or monitoring of a neuropsychiatric disorder or for enhancement of a sightline communication ability, and/or enhancement of a social sightline control ability.

In an A-nineteenth aspect of the present disclosure, in the sightline-position determination device, the neuropsychiatric disorder is included in anxiety disorders or neurodevelopmental disorders.

A sightline-position determination system in an A-twentieth aspect of the present disclosure includes the sightline-position determination device in any of the aforementioned aspects; and a sightline-position acquisition unit which includes a sightline direction acquisition unit configured to detect a direction of a sightline of the user, the sightline-position acquisition unit being configured to output the direction of the sightline of the user which has been detected by the sightline direction acquisition unit, to the sightline-position input unit.

In an A-twenty-first aspect of the present disclosure, in the sightline-position determination system, the sightline-position acquisition unit further includes a field-of-view image acquisition unit configured to acquire a field-of-view image of a field of view of the user, the field-of-view image acquisition unit being configured to acquire a position of the sightline of the user based on the field-of-view image.

In an A-twenty-second aspect of the present disclosure, in the sightline-position determination system, the sightline-position input unit includes a display position detection unit configured to detect a position of a presentation image presented to the user in the field-of-view image acquired by the field-of-view image acquisition unit, and a sightline-position transformation unit configured to transform a coordinate system of the field-of-view image into a coordinate system of the presentation image, based on the detected position of the presentation image.

A sightline-position determination method in an A-twenty-third aspect of the present disclosure includes: inputting a position of a sightline of a user; setting a determination region; determining whether or not the inputted position of the sightline falls within the determination region; and outputting feedback to the user when it is determined that the position of the sightline falls within the determination region.

In an A-twenty-fourth aspect of the present disclosure, the sightline-position determination method further includes measuring a first time having elapsed in a state where the position of the sightline remains in the determination region since the position of the sightline got to be located in the determination region, and determining that the position of the sightline falls within the determination region, when the measured first time has reached a preset maintaining time.

In an A-twenty-fifth aspect of the present disclosure, the sightline-position determination method includes measuring an index of a social sightline control ability, in addition to the first time.

In an A-twenty-sixth aspect of the present disclosure, in the sightline-position determination method, the index of the social sightline control ability is at least one of an analyzed value of the first time, a second time having elapsed since the position of the sightline was inputted first to the sightline-position input unit until the position of the sightline is determined to fall within the determination region, a distance between the position of the sightline of the user and the determination region, and a number of times it has been determined that the position of the sightline falls within the determination region.

In an A-twenty-seventh aspect of the present disclosure, in the sightline-position determination method, the feedback is outputted to the user in real-time.

In an A-twenty-eighth aspect of the present disclosure, the sightline-position determination method further includes setting a target position to which the user directs a sightline, and setting the determination region including the target position.

In an A-twenty-ninth aspect of the present disclosure, in the sightline-position determination method, the determination region is set such that the determination region includes one selected from an entire body of an animal, which includes a person, facing the user, a part of the body of the animal facing the user, an entire face of the animal facing the user, a part of the face of the animal facing the user, an eye part of the animal facing the user, and a part of an eyeball position of the animal facing the user.

In an A-thirtieth aspect of the present disclosure, in the sightline-position determination method, the determination region is set such that the determination region includes one selected from an entire body of an animal including a person, a part of the body of the animal, an entire face of the animal, a part of the face of the animal, an eye part of the animal, and an eyeball position of the animal, in an image of the animal displayed on a display facing the user.

In an A-thirty-first aspect of the present disclosure, in the sightline-position determination method, a size of the determination region is set based on a distance between left and right eyeball positions of an animal including a person facing the user.

In an A-thirty-second aspect of the present disclosure, in the sightline-position determination method, a lower limit value is set to the size of the determination region.

In an A-thirty-third aspect of the present disclosure, in the sightline-position determination method, the animal is a human.

In an A-thirty-fourth aspect of the present disclosure, in the sightline-position determination method, the determination as to whether or not the inputted position of the sightline falls within the set determination region is performed with a set difficulty level of training constituted by a plurality of tasks.

In an A-thirty-fifth aspect of the present disclosure, in the sightline-position determination method, when a first time having elapsed in a state where the position of the sightline remains in the determination region since the position of the sightline got to be located in the determination region has reached a preset maintaining time, and it is determined that the position of the sightline falls within the determination region, the difficulty level of the training is set, by setting a size of the determination region and/or a length of the maintaining time.

In an A-thirty-sixth aspect of the present disclosure, in the sightline-position determination method, the difficulty level of the training is set based on a result of determination as to whether or not the position of the sightline falls within the set determination region.

In an A-thirty-seventh aspect of the present disclosure, the sightline-position determination method includes successively presenting to the user a plurality of images each including the determination region set therein.

A training system in an A-thirty-eighth aspect of the present disclosure includes the sightline-position determination device in any of the aforementioned aspects or the sightline-position determination system in any of the aforementioned aspects.

A method for evaluating a sightline communication ability in an A-thirty-ninth aspect of the present disclosure uses the sightline-position determination method in any of the aforementioned aspects.

In an A-fortieth aspect of the present disclosure, in the method for evaluating a sightline communication ability, the sightline communication ability is a social sightline control ability or a social sightline transmission ability.

In an A-forty-first aspect of the present disclosure, in the method for evaluating a sightline communication ability, the social sightline control ability is a social sightline coincidence ability, a social sightline shifting ability, or a conjugate gaze ability.

A training method in an A-forty-second aspect of the present disclosure includes executing training using the sightline-position determination method in any of the aforementioned aspects.

In an A-forty-third aspect of the present disclosure, in the training method, the training is constituted by a plurality of tasks structured to obtain at least one result of determination as to whether or not the position of the sightline falls within the determination region, and the plurality of tasks are successively executed, and the tasks include directing a sightline to one of a plurality of the determination regions having different sizes, according to a teaching.

In an A-forty fourth aspect of the present disclosure, in the training method, the tasks include a first task in which the determination region is set such that the determination region includes a constituent part of a social object to which a sightline is to be directed; and a second task in which the determination region is set such that the determination region includes a constituent unit encompassing the constituent part.

In an A-forty-fifth aspect of the present disclosure, in the training method, the tasks include a third task in which the determination region is set such that the determination region includes the constituent part and the constituent unit, alternately.

In an A-forty-sixth aspect of the present disclosure, in the training method, the tasks include a fourth task of estimating a facial expression and/or an emotion of a social object in which the determination region is set, in addition to directing a sightline to the determination region.

In an A-forty-seventh aspect of the present disclosure, in the training method, a difficulty level of the training is set by a difficulty level of the tasks.

In an A-forty-eighth aspect of the present disclosure, in the training method, a difficulty level of the training is set based on an index including subjective or objective evaluation of the user.

In an A-forty-ninth aspect of the present disclosure, in the training method, the subjective evaluation of the user includes an evaluation, which is self-reported by the user, of hardship which was felt actually by the user executing the training.

In an A-fiftieth aspect of the present disclosure, in the training method, the objective evaluation of the user includes at least one of a result of the training, a heart rate change of the user during the training, and a change of a degree of opening of pupils of the user.

In an A-fifty first aspect of the present disclosure, in the training method, a difficulty level of the training is set by a difficulty level of sightline coincidence determination which is determination as to whether or not the inputted position of the sightline falls within the determination region.

In an A-fifty-second aspect of the present disclosure, the training method includes measuring an index of the social sightline control ability of the user and setting a difficulty level of the sightline coincidence determination based on the index.

A monitoring method in an A-fifty-third aspect of the present disclosure uses the sightline-position determination method in any of the aforementioned aspects.

A method for treating a psychiatric disorder in an A-fifty-fourth aspect of the present disclosure includes executing training using the training method in any of the aforementioned aspects.

In an A-fifty-fifth aspect of the present disclosure, in the method for treating a psychiatric disorder, the training is executed for 1 minute to 240 minutes per once, at a frequency of once per 90 days to 12 times per day, within a time period of 1 day to 1 year.

In an A-fifty-sixth aspect of the present disclosure, in the method for treating a psychiatric disorder, a difficulty level of the training is increased stepwisely.

In an A-fifty-seventh aspect of the present disclosure, in the method for treating a psychiatric disorder, the difficulty level of the training is changed depending on the user.

A program in an A-fifty-eighth aspect of the present disclosure causes a computer to execute the sightline-position determination method in any of the aforementioned aspects.

In an A-fifty-ninth aspect of the present disclosure, the program causes a computer to execute the training method in any of the aforementioned aspects.

Incidentally, arbitrary embodiments or modifications among the aforementioned various embodiments or modifications can be appropriately combined to provide the effects of the respective embodiments or modifications. Furthermore, embodiments can be combined with each other, examples can be combined with each other, or embodiments and examples can be combined with each other, and, also, features in different embodiments or different examples can be combined with each other.

### REFERENCE SIGNS LIST

1. sightline-position determination system
10. sightline-position determination device
11. sightline-position input unit
111. display position detection unit
112. sightline-position transformation unit
12. determination unit
13. output unit
14. target position setting unit
15. determination region setting unit
16. measurement unit
17. marker creation unit
20. sightline-position acquisition unit
21. image pickup unit
22. sightline direction acquisition unit
31. head mounted display
32. display
33. screen
34. presentation-image acquisition unit
35. wearable terminal
36. speaker
40. field-of-view image
50. difficulty level setting unit
51. manipulation unit
52. storage unit
53. control ability acquisition unit
54. maintaining-time setting unit
100. image of interest
P1. target position
P2. determination region

## Claims

1. A sightline-position determination device, comprising:
a sightline-position input unit to which a position of a sightline of a user is inputted;
a determination region setting unit which sets a determination region;
a determination unit which determines whether or not the position of the sightline inputted from the sightline-position input unit falls within the determination region; and
an output unit which outputs feedback to the user when the determination unit has determined that the position of the sightline falls within the determination region.

2. The sightline-position determination device according to claim 1, further comprising
a measurement unit which measures a first time having elapsed in a state where the position of the sightline remains in the determination region since the position of the sightline got to be located in the determination region, wherein
the determination unit determines that the position of the sightline falls within the determination region, when the first time measured by the measurement unit has reached a preset maintaining time.

3. The sightline-position determination device according to claim 1 or 2, wherein
the output unit outputs the feedback to the user in real-time.

4. The sightline-position determination device according to any one of claims 1 to 3, further comprising
a target position setting unit which sets a target position to which the user directs a sightline.

5. The sightline-position determination device according to any one of claims 1 to 4, wherein
the determination region setting unit sets the determination region such that the determination region includes one selected from an entire body of an animal, which includes a person, facing the user, a part of the body of the animal facing the user, an entire face of the animal facing the user, a part of the face of the animal facing the user, an eye part of the animal facing the user, and a part of an eyeball position of the animal facing the user.

6. The sightline-position determination device any one of claims 1 to 5, wherein
the determination region setting unit sets the determination region such that the determination region includes one selected from an entire body of an animal including a person, a part of the body of the animal, an entire face of the animal, a part of the face of the animal, an eye part of the animal, and an eyeball position of the animal, in an image of the animal displayed on a display facing the user.

7. The sightline-position determination device according to claim 5 or 6, wherein
the determination region setting unit sets the determination region such that the determination region includes an eyeball position of the animal.

8. The sightline-position determination device according to any one of claims 1 to 7, wherein
the determination region setting unit sets a size of the determination region, based on a distance between left and right eyeball positions of an animal, in an image of the animal, which includes a person, included in a field-of-view image of the user.

9. The sightline-position determination device according to claim 8, wherein
a lower limit value is set to the size of the determination region.

10. The sightline-position determination device according to any one of claims 5 to 9, wherein
the animal is a human.

11. The sightline-position determination device according to any one of claims 1 to 10, further comprising
a measurement unit which measures a first time having elapsed in a state where the position of the sightline remains in the determination region since the position of the sightline got to be located in the determination region, wherein
the measurement unit measures an index of a social sightline control ability of the user, in addition to the first time having elapsed in the state where the position of the sightline remains in the determination region since the position of the sightline got to be located in the determination region.

12. The sightline-position determination device according to claim 11, wherein
the index of the social sightline control ability is at least one of an analyzed value of the first time, a second time having elapsed since the position of the sightline was inputted first to the sightline-position input unit until the position of the sightline is determined to fall within the determination region, a distance between the position of the sightline of the user and the determination region, and a number of times it has been determined that the position of the sightline falls within the determination region.

13. The sightline-position determination device according to claim 11 or 12, wherein
the sightline-position determination device estimates a task which is difficult for the user, out of a plurality of tasks in training constituted by the plurality of tasks, by comparing the measured index of the social sightline control ability of the user with the index of the social sightline control ability of another user.

14. The sightline-position determination device according to any one of claims 1 to 10, further comprising
a difficulty level setting unit which sets a load of training constituted by a plurality of tasks.

15. The sightline-position determination device according to claim 14, wherein
the difficulty level setting unit sets the load of the training based on a result of determination by the determination unit.

16. The sightline-position determination device according to claim 14 or 15, further comprising
a measurement unit which measures a first time having elapsed in a state where the position of the sightline remains in the determination region since the position of the sightline got to be located in the determination region, wherein
the determination unit determines that the position of the sightline falls within in the determination region, when the first time measured by the measurement unit has reached a preset maintaining time, and
the difficulty level setting unit sets the load of the training, by setting a size of the determination region and/or a length of the maintaining time.

17. The sightline-position determination device according to any one of claims 14 to 16, further comprising
a measurement unit which measures a first time having elapsed in a state where the position of the sightline remains in the determination region since the position of the sightline got to be located in the determination region, wherein
the measurement unit measures an index of the social sightline control ability of the user, in addition to the first time.

18. The sightline-position determination device according to claim 17, wherein
the index of the social sightline control ability is at least one of an analyzed value of the first time, a second time having elapsed since the position of the sightline was inputted first to the sightline-position input unit until the position of the sightline is determined to fall within the determination region, a distance between the position of the sightline of the user and the determination region, and a number of times it has been determined that the position of the sightline falls within the determination region.

19. The sightline-position determination device according to claim 17 or 18, wherein
the difficulty level setting unit increases the load of the training, when a value of the measured index of the social sightline control ability is greater than a value of the index of the social sightline control ability which was measured before the measured index of the social sightline control ability.

20. The sightline-position determination device according to any one of claims 17 to 19, wherein
the difficulty level setting unit estimates the task which is difficult for the user, by making a comparison between a deviation of the measured index of the social sightline control ability of the user in each of the tasks with respect to an average value of the measured index of the social sightline control ability of the user, and a deviation of the index of the social sightline control ability of another user in each of the tasks with respect to an average value of the index of the social sightline control ability of the another user, and
the difficulty level setting unit sets the load of the training based on the estimated task.

21. The sightline-position determination device according to any one of claims 14 to 20, further comprising
a manipulation unit capable of receiving a difficulty level changing manipulation for changing the load of the training, wherein
the difficulty level setting unit sets the difficulty level of the training based on the difficulty level changing manipulation received by the manipulation unit.

22. The sightline-position determination device according to any one of claims 1 to 21, wherein
the feedback is performed using one or more senses selected from a visual sense, an auditory sense, and a tactile sense.

23. The sightline-position determination device according to any one of claims 1 to 22, wherein
the sightline-position determination device is used for diagnosis, severity classification, treatment, training and/or monitoring of a neuropsychiatric disorder or for enhancement of a sightline communication ability.

24. The sightline-position determination device according to claim 23, wherein
the neuropsychiatric disorder is anxiety disorders or neurodevelopmental disorders.

25. A sightline-position determination system, comprising:
the sightline-position determination device according to any one of claims 1 to 24; and
a sightline-position acquisition unit which includes a sightline direction acquisition unit which detects a direction of a sightline of the user, the sightline-position acquisition unit outputting the direction of the sightline of the user which has been detected by the sightline direction acquisition unit, to the sightline-position input unit.

26. The sightline-position determination system according to claim 25, wherein
the sightline-position acquisition unit further includes a field-of-view image acquisition unit which acquires a field-of-view image of the user, the sightline-position acquisition acquiring a position of the sightline of the user based on the field-of-view image.

27. The sightline-position determination system according to claim 26, wherein
the sightline-position input unit includes
a display position detection unit which detects a position of a presentation image presented to the user in the field-of-view image acquired by the field-of-view image acquisition unit, and
a sightline-position transformation unit which transforms the position of the sightline of the user in a coordinate system of the field-of-view image into a position of the sightline of the user in a coordinate system of the presentation image, based on the detected position of the presentation image.

28. A sightline-position determination method, comprising;
inputting a position of a sightline of a user;
setting a determination region;
determining whether or not the inputted position of the sightline falls within the determination region; and
outputting feedback to the user when it is determined that the position of the sightline falls within the determination region.

29. The sightline-position determination method according to claim 28, further comprising
measuring a first time having elapsed in a state where the position of the sightline remains in the determination region since the position of the sightline got to be located in the determination region, and
determining that the position of the sightline falls within the determination region, when the measured first time has reached a preset maintaining time.

30. The sightline-position determination method according to claim 28 or 29, wherein
the feedback id outputted to the user in real-time.

31. The sightline-position determination method according to any one of claims 28 to 30, further comprising
setting a target position to which the user directs s sightline, and
setting the determination region including the target position.

32. The sightline-position determination method according to any one of claims 28 to 31, wherein
the determination region is set such that the determination region includes one selected from an entire body of an animal, which includes a person, facing the user, a part of the body of the animal facing the user, an entire face of the animal facing the user, a part of the face of the animal facing the user, an eye part of the animal facing the user, and a part of an eyeball position of the animal facing the user

33. The sightline-position determination method according to any one of claims 28 to 31, wherein
the determination region is set such that the determination region includes one selected from an entire body of an animal including a person, a part of the body of the animal, an entire face of the animal, a part of the face of the animal, an eye part of the animal, and an eyeball position of the animal, in an image of the animal displayed on a display facing the user.

34. The sightline-position determination method any one of claims 28 to 33, wherein
a size of the determination region is set based on a distance between left and right eyeball positions of an animal including a person facing the user.

35. The sightline-position determination method according to claim 34, wherein
a lower limit value is set to the size of the determination region.

36. The sightline-position determination method according to claim 35, wherein
the lower limit value is determined based on sightline-position variation data representing a sightline position which varies with time when the user gazes at an arbitrary point.

37. The sightline-position determination method according to any one of claims 32 to 36, wherein
the animal is a human.

38. A training system, comprising:
the sightline-position determination device according to any one of claims 1 to 24 or the sightline-position determination system according to any one of claims 25 to 27.

39. An evaluating method of a sightline communication ability, comprising
using the sightline-position determination method according to any one of claims 28 to 37.

40. The evaluating method according to claim 39, wherein
the sightline communication ability is a social sightline control ability or a social sightline transmission ability.

41. The evaluating method according to claim 40, wherein
the social sightline control ability is a social sightline coincidence ability, a social sightline shifting ability, or a conjugate gaze ability.

42. A training method, comprising
executing training using the sightline-position determination method according to any one of claims 28 to 37.

43. The training method according to claim 42, wherein
the training is constituted by a plurality of tasks structured to obtain at least one result of determination as to whether or not the position of the sightline falls within the determination region, and the plurality of tasks are successively executed, and
the tasks include directing a sightline to one of a plurality of the determination regions, according to a teaching.

44. The training method according to claim 43, wherein
the tasks include a first task in which the determination region is set such that the determination region includes a constituent part of a social object to which a sightline is to be directed; and a second task in which the determination region is set such that the determination region includes a constituent unit encompassing the constituent part.

45. The training method according to claim 44, wherein
the tasks include a third task in which the determination region is set such that the determination region includes the constituent part and the constituent unit, alternately.

46. The training method according to any one of claims 43 to 45, wherein
the tasks include a fourth task of estimating a facial expression and/or an emotion of a social object in which the determination region is set, in addition to directing a sightline to the determination region.

47. The training method according to any one of claims 43 to 46, wherein
a load of the training is set according to a result of determination as to whether or not the position of the sightline falls within the determination region.

48. The training method according to claim 47, wherein
when a first time having elapsed in a state where the position of the sightline remains in the determination region since the position of the sightline got to be located in the determination region has reached a preset maintaining time, and it is determined that the position of the sightline falls within in the determination region, the load of the training is set by setting a size of the determination region and/or a length of the maintaining time.

49. The training method according to any one of claims 43 to 48, wherein
a load of the training is set by a difficulty level of the tasks.

50. The training method according to any one of claims 43 to 49, wherein
a load of the training is set based on an index including subjective or objective evaluation of the user.

51. The training method according to claim 50, wherein
the subjective evaluation of the user includes an evaluation, which is self-reported by the user, of hardship which was felt actually by the user executing the training.

52. The training method according to claim 50 or 51, wherein
the objective evaluation of the user includes at least one of a result of the training, a heart rate change and/or a heart-rate variation index of the user during the training, a degree of opening of pupils of the user, and a change of a blinking frequency of the user.

53. The training method according to any one of claims 42 to 52, comprising
measuring a first time having elapsed in a state where the position of the sightline remains in the determination region since the position of the sightline got to be located in the determination region, and an index of a social sightline control ability which is an ability to control and use a sightline as a signal.

54. The training method according to claim 53, wherein
the index of the social sightline control ability is at least one of an analyzed value of the first time, a second time having elapsed since the position of the sightline was inputted first until the position of the sightline is determined to fall within the determination region, a distance between the position of the sightline of the user and the determination region, and a number of times it has been determined that the position of the sightline falls within the determination region.

55. The training method according to claim 53 or 54, comprising
increasing a load of the training, when a value of the measured index of the social sightline control ability is greater than a value of the index of the social sightline control ability which was measured before the measured index of the social sightline control ability.

56. The training method according to any one of claims 53 to 55, comprising
estimating the task which is difficult for the user, by making a comparison between a deviation of the measured index of the social sightline control ability of the user in each of the tasks with respect to an average value of the measured index of the social sightline control ability of the user, and a deviation of the index of the social sightline control ability of another user in each of the tasks with respect to an average value of the index of the social sightline control ability of the another user, and
setting a load of the training based on the estimated task.

57. The training method according to any one of claims 42 to 55, comprising
setting a difficulty level of the training based on a difficulty level changing manipulation for changing the load of the training.

58. The training method according to any one of claims 42 to 57, wherein
the training is structured in such a way as to present, to the user, tasks successively in ascending order of a difficulty level, out of a plurality of tasks structured to obtain at least one result of determination as to whether or not the position of the sightline falls within the determination region.

59. A monitoring method, comprising
using the sightline-position determination method according to any one of claims 28 to 37.

60. A method for treating a neuropsychiatric disorder, comprising
executing training using the training method according to any one of claims 42 to 58.

61. The method for treating a neuropsychiatric disorder according to claim 60, wherein
the training is executed for 1 minute to 240 minutes per once, at a frequency of once per 90 days to 12 times per day, within a time period of 1 day to 1 year.

62. The method for treating a neuropsychiatric disorder according to claim 60 or 61, wherein
the load of the training is increased stepwisely.

63. The method for treating a neuropsychiatric disorder according to any one of claims 60 to 62, wherein
a difficulty level of the training is changed depending on the user.

64. A method for diagnosing a neuropsychiatric disorder, comprising
using the sightline-position determination method according to any one of claims 28 to 37.

65. A program for causing a computer to execute the sightline-position determination method according to any one of claims 28 to 37.

66. A program for causing a computer to execute the training method according to any one of claims 42 to 58.
